(19) Europäisches Patentamt
European Patent Office
Office européen des brevets

(11) Veröffentlichungsnummer: **0 173 317**
**A1**

# (12) EUROPÄISCHE PATENTANMELDUNG

(21) Anmeldenummer: **85110828.2**

(22) Anmeldetag: **19.08.85**

(51) Int. Cl.⁴: **C 07 D 239/42**, A 01 N 47/44

(30) Priorität: **30.08.84 DE 3431915**
**28.05.85 DE 3519091**

(43) Veröffentlichungstag der Anmeldung: **05.03.86**
**Patentblatt 86/10**

(84) Benannte Vertragsstaaten: **AT BE CH DE FR GB IT LI NL SE**

(71) Anmelder: **BAYER AG, Konzernverwaltung RP**
**Patentabteilung, D-5090 Leverkusen 1 Bayerwerk (DE)**

(72) Erfinder: **Diehr, Hans-Joachim, Dr., Höhe 35,**
**D-5600 Wuppertal 1 (DE)**
Erfinder: **Fest, Christa, Dr., Im Johannistal 20,**
**D-5600 Wuppertal 1 (DE)**
Erfinder: **Kirsten, Rolf, Dr., Carl-Langhans-Strasse 27,**
**D-4019 Monheim (DE)**
Erfinder: **Kluth, Joachim, Dr.,**
**Kurt-Schumacher-Strasse 9, D-4018 Langenfeld (DE)**
Erfinder: **Müller, Klaus-Helmut, Dr., Bockhackstrasse 55,**
**D-4000 Düsseldorf 13 (DE)**
Erfinder: **Pfister, Theodor, Dr., Lichtenberger Strasse 30,**
**D-4019 Monheim (DE)**
Erfinder: **Priesnitz, Uwe, Dr., Severinstrasse 58,**
**D-5650 Solingen 1 (DE)**
Erfinder: **Riebel, Hans-Jochem, Dr., In der Beek 92,**
**D-5600 Wuppertal 1 (DE)**
Erfinder: **Roy, Wolfgang, Dr., Walter-Kolb-Strasse 47,**
**D-4018 Langenfeld (DE)**
Erfinder: **Santel, Hans-Joachim, Dr., Gerstenkamp 19,**
**D-5000 Köln 80 (DE)**
Erfinder: **Schmidt, Robert R., Dr., Im Waldwinkel 110,**
**D-5060 Bergisch Gladbach 2 (DE)**

(54) **Unsymmetrische Sulfonylguanidine.**

(57) Die Erfindung betrifft neue unsymmetrische Sulfonylguanidine der allgemeinen Formel (I)

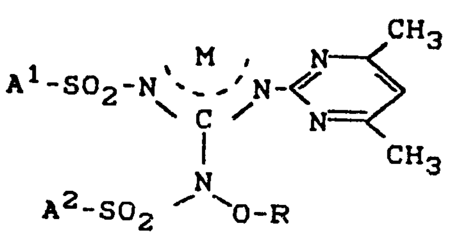

(I)

(worin die Reste $A^1$, $A^2$, R und M die in der Beschreibung angegebenen Bedeutungen haben), sowie 1:1-Addukte von Verbindungen der Formel (I) mit starken Säuren, Verfahren zu ihrer Herstellung und ihre Verwendung als Herbizide.

EP 0 173 317 A1

BAYER AKTIENGESELLSCHAFT
Konzernverwaltung RP
Patentabteilung

5090 Leverkusen, Bayerwerk
Bi/bo/c
Ib

Unsymmetrische Sulfonylguanidine

Die Erfindung betrifft neue unsymmetrische Sulfonylguanidine, Verfahren zu ihrer Herstellung und ihre Verwendung als Herbizide.

Verschiedene Guanidin-Derivate sind aus Patentschriften (vgl. DE-AS 1 089 210, DD-PS 71 016 und 84 530) als potentielle Herbizide bekannt geworden, haben jedoch bisher als Mittel zur Unkrautbekämpfung und/oder Regulierung des Pflanzenwachstums keine größere Bedeutung erlangt. Weitere Guanidin-Derivate sind bekannt (vgl. DE-OS 3 334 455).

Es wurden nun neue unsymmetrische Sulfonylguanidine der allgemeinen Formel (I)

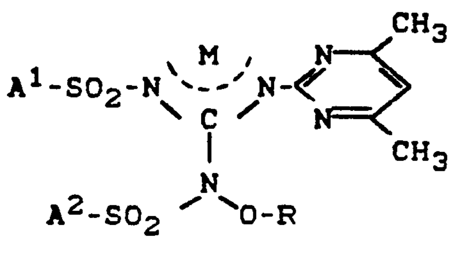

(I)

in welcher

die Reste $A^1$ und $A^2$ in jedem Einzelfall verschieden sind und jeweils einer der beiden Reste ($A^1$ oder $A^2$) für einen Rest der Formel

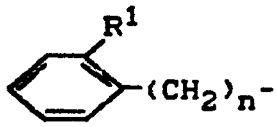

steht, worin

n für Null oder 1 steht und

$R^1$ für Halogen, Cyano, Nitro, $C_1$-$C_4$-Alkyl [welches gegebenenfalls durch Fluor, Chlor, Brom, Cyano, $C_1$-$C_4$-Alkoxy-carbonyl, $C_1$-$C_4$-Alkylamino-carbonyl, Di-($C_1$-$C_4$-alkyl)-amino-carbonyl, $C_1$-$C_4$-Alkoxy, $C_1$-$C_4$-Alkylthio, $C_1$-$C_4$-Alkylsulfinyl oder $C_1$-$C_4$-Alkyl-sulfonyl substituiert ist], für $C_1$-$C_4$-Alkoxy [welches gegebenenfalls durch Fluor, Chlor, Brom, Cyano, $C_1$-$C_4$-Alkoxy-carbonyl, $C_1$-$C_4$-Alkoxy, $C_1$-$C_4$-Alkylthio, $C_1$-$C_4$-Alkyl-sulfinyl oder $C_1$-$C_4$-Alkyl-sulfonyl substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor, Chlor, Brom, Cyano oder $C_1$-$C_4$-Alkoxy-carbonyl substituiert sind], für $C_1$-$C_4$-Alkylamino-sulfonyl, Di-($C_1$-$C_4$-alkyl)-amino-sulfonyl, $C_1$-$C_4$-Alkoxyamino-sulfonyl, Benzyloxy-aminosulfonyl, $C_1$-$C_4$-Alkoxy-$C_1$-$C_4$-alkyl-amino-sulfonyl, für Phenyl oder Phenoxy, für

$C_1$-$C_4$-Akoxysulfonyl, für $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], für $C_2$-$C_6$-Alkinyl [welches gegebenenfalls durch Fluor, Chlor, Brom, Cyano oder $C_1$-$C_4$-Alkoxy-carbonyl substituiert ist], für $C_3$-$C_6$-Alkenoxy [welches gegebenenfalls durch Fluor, Chlor, Brom, Cyano oder $C_1$-$C_4$-Alkoxy-carbonyl substituiert ist], für $C_3$-$C_6$-Alkenylthio [welches gegebenenfalls durch Fluor, Chlor, Brom, Cyano oder $C_1$-$C_4$-Alkoxy-carbonyl substituiert ist], für $C_1$-$C_6$-Alkoxy-carbonyl [welches gegebenenfalls durch Fluor, Chlor, Brom, Cyano oder $C_1$-$C_4$-Alkoxy substituiert ist], für $C_3$-$C_6$-Cycloalkyloxy-carbonyl, für $C_1$-$C_6$-Alkylthio-carbonyl [welches gegebenenfalls durch Fluor, Chlor, Brom, Cyano oder $C_1$-$C_4$-Alkoxy-carbonyl substituiert ist], für Aminocarbonyl, $C_1$-$C_4$-Alkylamino-carbonyl, Di-($C_1$-$C_4$-alkyl)-amino-carbonyl, $C_1$-$C_4$-Alkoxyamino-carbonyl, $C_3$-$C_4$-Alkenoxyamino-carbonyl, Benzyloxyamino-carbonyl, $C_1$-$C_4$-Alkoxy-$C_1$-$C_4$-alkyl-amino-carbonyl, Dimethylhydrazino-carbonyl, oder Dimethylhydrazino-sulfonyl steht,

und in welcher jeweils der andere der beiden Reste ($A^2$ bzw. $A^1$)

für $C_1$-$C_{12}$-Alkyl [welches gegebenenfalls durch Fluor, Chlor, Brom, Cyano, Nitro, $C_1$-$C_4$-Alkoxy-carbonyl, $C_1$-$C_4$-Alkylamino-carbonyl, Di-($C_1$-$C_4$-alkyl)-amino-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist] für

$C_1$-$C_{12}$-Alkoxy [welches gegebenenfalls durch Fluor, Chlor, Brom, Cyano, Nitro, $C_1$-$C_4$-Alkoxycarbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_2$-$C_{12}$-Alkenyl [welches gegebenenfalls durch Fluor, Chlor, Brom, Cyano, Nitro, $C_1$-$C_4$-Alkoxy, $C_1$-$C_4$-Alkylthio oder Phenyl substituiert ist], für $C_2$-$C_{12}$-Alkenyloxy [welches gegebenenfalls durch Fluor, Chlor, Brom, Cyano, Nitro, $C_1$-$C_4$-Alkoxy, $C_1$-$C_4$-Alkylthio oder Phenyl substituiert ist], für $C_1$-$C_8$-Alkylamino, $C_3$-$C_6$-Cycloalkylamino, Di-($C_3$-$C_6$-cycloalkyl)-amino oder Di-($C_1$-$C_4$-alkyl)-amino steht [welche gegebenenfalls durch Fluor, Chlor, Brom, Cyano, Nitro, Phenyl, Phenoxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiert sind], für $C_2$-$C_8$-Alkenylamino [welches gegebenenfalls durch Fluor, Chlor, Brom, Cyano, Nitro oder Phenyl substituiert ist], für Phenylamino oder Benzylamino [welche gegebenenfalls durch Fluor, Chlor, Brom, $C_1$-$C_4$-Alkyl, Trifluormethyl, $C_1$-$C_4$-Alkoxy, $C_1$-$C_2$-Fluoralkoxy, $C_1$-$C_4$-Alkylthio, $C_1$-$C_4$-Alkylsulfinyl, $C_1$-$C_4$-Alkylsulfonyl, Cyano, Nitro und/oder $C_1$-$C_4$-Alkoxy-carbonyl substituiert sind], für Benzyl oder Phenyl steht, wobei letztere gegebenenfalls durch einen oder mehrere Reste aus der Reihe Halogen, Cyano, Nitro, $C_1$-$C_4$-Alkyl [welches gegebenenfalls durch Fluor, Chlor, Brom, Cyano, Carboxy, $C_1$-$C_4$-Alkoxy-carbonyl, $C_1$-$C_4$-Alkyl-amino-carbonyl, $C_1$-$C_4$-Alkoxy-amino-carbonyl, Di-($C_1$-$C_4$-alkyl)-amino-carbonyl, $C_1$-$C_4$-Alkoxy, Formyloxy, $C_1$-$C_4$-Alkyl-carbonyloxy, $C_1$-$C_4$-Alkoxy-

carbonyloxy, $C_1$-$C_4$-Alkyl-amino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl oder Phenyl substituiert ist], $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], $C_2$-$C_6$-Alkinyl [welches gegebenenfalls durch Fluor, Chlor, Brom, Cyano, $C_1$-$C_4$-Alkoxy-carbonyl oder Phenyl substituiert ist], $C_1$-$C_4$-Alkoxy [welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist], $C_3$-$C_6$-Alkenoxy [welches gegebenenfalls durch Fluor, Chlor, Brom, Cyano oder $C_1$-$C_4$-Alkoxy-carbonyl substituiert ist], $C_3$-$C_6$-Alkenylthio [welches gegebenenfalls durch Fluor, Chlor, Brom, Cyano oder $C_1$-$C_4$-Alkoxy-carbonyl substituiert ist], $C_1$-$C_4$-Alkylthio, $C_1$-$C_4$-Alkylsulfinyl oder $C_1$-$C_4$-Alkylsulfonyl [welche gegebenenfalls durch Fluor, Chlor, Brom, Cyano oder $C_1$-$C_4$-Alkoxy-carbonyl substituiert sind], Phenyl, Phenoxy, Di-($C_1$-$C_4$-alkyl)-amino-sulfonyl, $C_1$-$C_4$-Alkyl-amino-sulfonyl, $C_1$-$C_4$-Alkoxy-$C_1$-$C_4$-alkyl-amino-sulfonyl, $C_1$-$C_4$-Alkoxy-sulfonyl, $C_1$-$C_4$-Alkoxy-amino-sulfonyl, $C_1$-$C_6$-Alkoxy-carbonyl, $C_3$-$C_6$-Cycloalkoxy-carbonyl, $C_1$-$C_6$-Alkylamino-carbonyl, Di-($C_1$-$C_4$-alkyl)-amino-carbonyl, $C_1$-$C_6$-Alkoxy-amino-carbonyl, $C_1$-$C_4$-Alkoxy-$C_1$-$C_4$-alkyl-amino-carbonyl, $C_1$-$C_6$-Alkylaminosulfonylamino oder Di-($C_1$-$C_6$-alkyl)-amino-sulfonylamino substituiert sind und/oder gegebenenfalls benzanelliert sind:

in welcher weiter

R für $C_1$-$C_{12}$-Alkyl [welches gegebenenfalls durch Fluor, Chlor, $C_1$-$C_4$-Alkoxy, $C_1$-$C_4$-Alkylthio, $C_1$-$C_4$-Alkylsulfinyl, $C_1$-$C_4$-Alkylsulfonyl, Cyano oder Nitro substituiert ist], $C_3$-$C_6$-Alkenyl [welches gegebenenfalls durch Fluor oder Chlor substituiert ist], $C_3$-$C_6$-Alkinyl, $C_1$-$C_4$-Alkoxy-carbonyl-$C_1$-$C_2$-alkyl, Aminocarbonyl-$C_1$-$C_2$-alkyl, $C_1$-$C_4$-Alkyl-amino-carbonyl-$C_1$-$C_2$-alkyl, Di-($C_1$-$C_4$-alkyl)-amino-carbonyl-$C_1$-$C_2$-alkyl, für Phenyl, Phenylethyl, Benzhydryl oder Benzyl [welche gegebenenfalls durch Fluor, Chlor, Brom, Cyano, Nitro, $C_1$-$C_4$-Alkyl, $C_1$-$C_4$-Alkoxy, Carboxy oder $C_1$-$C_4$-Alkoxy-carbonyl substituiert sind] steht und

M für Wasserstoff oder ein Metalläquivalent steht,

sowie 1:1-Addukte von Verbindungen der Formel (I) mit starken Säuren gefunden,

wobei folgende Verbindungen ausgenommen sind:

N'-(4,6-Dimethyl-pyrimidin-2-yl)-N"-methoxy-N"-(2-chlor-benzolsulfonyl-N"'-(2-methoxycarbonyl-benzolsulfonyl)-guanidin,
N'-(4,6-Dimethyl-pyrimidin-2-yl)-N"-methoxy-N"-(3-chlor-benzolsulfonyl)-N"'-(2-methoxycarbonyl-benzolsulfonyl)-guanidin und
N'-(4,6-Dimethyl-pyrimidin-2-yl)-N"-methoxy-N"-(4-methyl-benzolsulfonyl)-N"'-(2-methoxycarbonyl-benzolsulfonyl)-guanidin.

Die allgemeine Formel (I) steht - wenn M für Wasserstoff steht - für die einzelnen Tautomeren der Formeln (IA) und (IB).

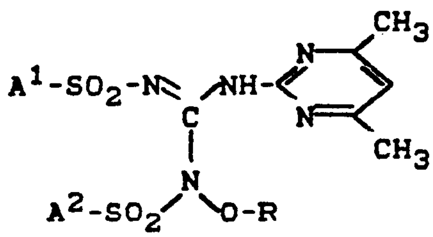

(IA)

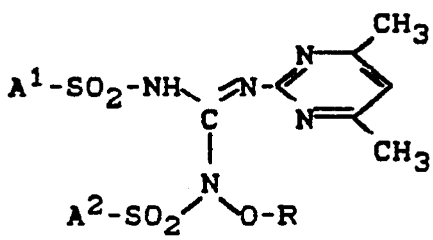

(IB)

in welchen

$A^1$, $A^2$ und R die oben angegebenen Bedeutungen haben, sowie für Gemische der Tautomeren (IA) und (IB).

Das Mischungsverhältnis (IA)/(IB) hängt von aggregationsbestimmenden Faktoren, wie z.B. Temperatur, Lösungsmittel und Konzentration ab.

Man erhält die neuen unsymmetrischen Sulfonylguanidine der Formel (I)

(a) für den Fall, daß M für Wasserstoff steht, wenn man Sulfonylguanidine der Formel (II)

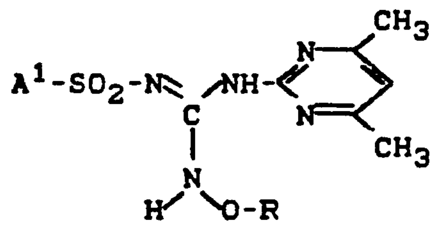

(II)

in welcher

$A^1$ und R die oben angegebenen Bedeutungen haben,

mit Sulfonsäurechloriden der Formel (III)

$$A^2-SO_2-Cl \qquad (III)$$

in welcher

$A^2$ die oben angegebene Bedeutung hat,

gegebenenfalls in Gegenwart von Säureakzeptoren und gegebenenfalls in Gegenwart von Verdünnungsmitteln umsetzt;

oder

(b) für den Fall, daß M für Wasserstoff steht, wenn man Sulfonylguanidine der Formel (IV)

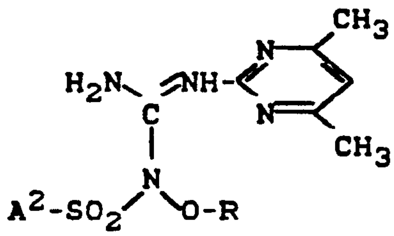

(IV)

in welcher

$A^2$ und R die oben angegebenen Bedeutungen haben,

mit Sulfonsäurechloriden der Formel (V)

$$A^1\text{-}SO_2Cl \qquad (V)$$

in welcher

$A^1$ die oben angegebene Bedeutung hat,

gegebenenfalls in Gegenwart von Säureakzeptoren und gegebenenfalls in Gegenwart von Verdünnungsmitteln umsetzt;

oder

(c) für den Fall, daß M für ein Metalläquivalent steht, wenn man die nach dem oben unter (a) und (b) angegebenen Verfahren erhältlichen Verbindungen der Formel (I), in welcher M für Wasserstoff steht und R sowie $A^1$ und $A^2$ die oben angegebenen Bedeutungen haben,

mit Metall-hydroxiden, -hydriden oder -alkanolaten oder mit metallorganischen Verbindungen, gegebenenfalls in Gegenwart von Verdünnungsmitteln umsetzt;

oder

(d) für den Fall, daß 1:1-Addukte von Verbindungen der Formel (I) mit starken Säuren herzustellen sind,

wenn man Verbindungen der Formel (I), in welcher M für Wasserstoff steht und R sowie $A^1$ und $A^2$ die oben angegebenen Bedeutungen haben,

mit starken Säuren gegebenenfalls in Gegenwart von inerten Verdünnungsmitteln umsetzt.

Die neuen unsymmetrischen Sulfonylguanidine der Formel (I) und ihre 1:1-Addukte mit starken Säuren zeichnen sich durch eine wesentlich bessere herbizide Wirksamkeit aus, als vorbekannte Guanidine gleicher Wirkungsrichtung.

Gegenstand der Erfindung sind vorzugsweise Verbindungen der Formel (I), in welcher

(1) $A^1$ für den Rest

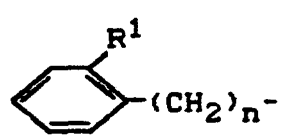 steht, worin

n für O steht,

$R^1$ für Fluor, Chlor, Brom, Cyano, Nitro, Methyl [welches gegebenenfalls durch Fluor, Chlor, Brom, Cyano, $C_1$-$C_3$-Alkoxy-carbonyl, $C_1$-$C_3$-Alkoxy, $C_1$-$C_3$-Alkylthio, $C_1$-$C_3$-Alkylsulfinyl oder $C_1$-$C_3$-Alkylsulfonyl substituiert ist], für Methoxy [welches gegebenenfalls durch Fluor, Chlor, Brom, Cyano, $C_1$-$C_3$-Alkoxy-carbonyl oder $C_1$-$C_3$-Alkoxy substituiert ist], für $C_1$-$C_3$-Alkylthio, für $C_1$-$C_3$-Alkylsulfinyl oder $C_1$-$C_3$-Alkylsulfonyl [welche gegebenenfalls durch Fluor, Chlor, Brom, Cyano oder $C_1$-$C_3$-Alkoxy-carbonyl substituiert sind], für $C_1$-$C_3$-Alkylamino-sulfonyl, Di-($C_1$-$C_3$-alkyl)-amino-sulfonyl, $C_1$-$C_3$-Alkoxy-amino-sulfonyl, $C_1$-$C_3$-Alkoxy-methyl-amino-sulfonyl, für Phenyl, für $C_1$-$C_3$-Alkoxy-sulfonyl, für $C_1$-$C_4$-Alkoxy-carbonyl, $C_1$-$C_4$-Alkylthio-carbonyl, $C_1$-$C_4$-Alkyl-amino-carbonyl, Di-($C_1$-$C_3$-alkyl)-amino-carbonyl, $C_1$-$C_4$-Alkoxyamino-carbonyl, $C_1$-$C_3$-Alkoxy-methyl-amino-carbonyl, Dimethylhydrazino-carbonyl oder Dimethylhydrazino-sulfonyl steht; in welcher weiter

$A^2$ für $C_1$-$C_8$-Alkyl [welches gegebenenfalls durch Fluor, Chlor oder Cyano substituiert ist], für $C_1$-$C_8$-Alkoxy [welches gegebenenfalls durch Fluor, Chlor oder Cyano substituiert ist], für $C_2$-$C_8$-Alkenyl [welches gegebenenfalls durch Fluor, Chlor,

Cyano oder Phenyl substituiert ist], für $C_2$-$C_8$-Alkenyloxy [welches gegebenenfalls durch Fluor, Chlor, Cyano oder Phenyl substituiert ist] für $C_1$-$C_6$-Alkylamino, $C_3$-$C_6$-Cycloalkylamino oder Di-($C_1$-$C_3$-alkyl)-amino [welche gegebenenfalls durch Fluor, Chlor, Cyano, Phenyl, Phenoxy oder $C_1$-$C_2$-Alkoxy substituiert sind], für $C_2$-$C_6$-Alkenylamino (welches gegebenenfalls durch Fluor, Chlor, Cyano oder Phenyl substituiert ist], für Phenylamino oder Benzylamino [welche gegebenenfalls durch Fluor, Chlor, Brom, $C_1$-$C_4$-Alkyl, Trifluormethyl, $C_1$-$C_3$-Alkoxy, $C_1$-$C_2$-Fluoralkoxy, $C_1$-$C_3$-Alkylthio, $C_1$-$C_3$-Alkylsulfinyl, $C_1$-$C_3$-Alkyl-sulfonyl, Cyano, Nitro und/oder $C_1$-$C_3$-Alkoxy-carbonyl substituiert sind], für Benzyl oder Phenyl steht, welche - mit der Maßgabe, daß $A^1$ und $A^2$ in jedem Einzelfall verschieden sind - gegebenenfalls durch einen oder mehrere Reste aus der Reihe Fluor, Chlor, Brom, Cyano, Nitro, Methyl [welches gegebenenfalls durch Fluor, Chlor, Brom, Cyano, $C_1$-$C_3$-Alkoxy-carbonyl, $C_1$-$C_3$-Alkyl-amino-carbonyl, Di-($C_1$-$C_3$-alkyl)-amino-carbonyl, $C_1$-$C_3$-Alkoxy, $C_1$-$C_3$-Alkylthio, $C_1$-$C_3$-Alkylsulfinyl, $C_1$-$C_3$-Alkylsulfonyl, Di-($C_1$-$C_3$-alkyl)-amino-sulfonyl oder Phenyl substituiert ist], Methoxy [welches gegebenenfalls durch Fluor, Chlor, Brom, Cyano oder $C_1$-$C_3$-Alkoxy-carbonyl substituiert ist], $C_1$-$C_3$-Alkylthio, $C_1$-$C_3$-Alkylsulfinyl oder $C_1$-$C_3$-Alkylsulfonyl [welche gegebenenfalls durch Fluor, Chlor, Brom, Cyano oder $C_1$-$C_3$-Alkoxy-carbonyl substituiert sind], Phenyl, Phenoxy, $C_1$-$C_3$-Alkyl-amino-sulfonyl,

Di-($C_1$-$C_3$-alkyl)-amino-sulfonyl, $C_1$-$C_3$-Alkoxy-amino-sulfonyl, $C_1$-$C_3$-Alkoxy-$C_1$-$C_3$-alkyl-amino-sulfonyl, $C_1$-$C_3$-Alkoxy-sulfonyl, $C_1$-$C_4$-Alkoxy-carbonyl, $C_1$-$C_3$-Alkyl-amino-carbonyl, Di-($C_1$-$C_3$-alkyl)-amino-carbonyl, $C_1$-$C_4$-Alkoxy-amino-carbonyl oder $C_1$-$C_3$-Alkoxy-$C_1$-$C_3$-alkyl-amino-carbonyl substituiert ist; in welcher weiter

M für Wasserstoff oder ein Natrium-, Kalium- oder Calcium-Äquivalent steht und

R für $C_1$-$C_8$-Alkyl [welches gegebenenfalls durch Fluor, Chlor oder Cyano substituiert ist], $C_3$-$C_5$-Alkenyl, $C_1$-$C_3$-Alkoxy-carbonyl-$C_1$-$C_2$-alkyl, Phenyl, Phenylethyl oder Benzyl [welches gegebenenfalls durch Fluor, Chlor, Brom, Cyano, Nitro, $C_1$-$C_3$-Alkyl, $C_1$-$C_3$-Alkoxy oder $C_1$-$C_3$-Alkoxy-carbonyl substituiert ist] steht;

oder in welcher

(2) $A^1$ die vorausgehend unter (1) vorzugsweise für $A^2$ angegebene Bedeutung hat und

$A^2$ die vorausgehend unter (1) vorzugsweise für $A^1$ angegebene Bedeutung hat
mit der Maßgabe, daß $A^1$ und $A^2$ verschieden sind und weiter

M und R die vorausgehend unter (1) vorzugsweise angegebenen Bedeutungen haben,

sowie 1:1-Addukte von Verbindungen der Formel (I) - wie vorausgehend unter (1) und (2) definiert - mit Halogenwasserstoffsäuren, wie Hydrogen-chlorid, -bromid und -iodid, mit Schwefelsäure, Trifluoressigsäure, mit gegebenenfalls durch Fluor oder Chlor substituierten Alkansulfonsäuren mit bis zu 4 Kohlenstoffatomen oder auch mit Benzol- oder Naphthalinsulfonsäuren, welche gegebenenfalls durch Fluor, Chlor, Brom oder Methyl substituiert sind, wobei die bei der allgemeinen Restedefinition der Formel (I) einzeln genannten Verbindungen ausgenommen sind.

Verwendet man beispielsweise für die Verfahrensvariante (a) N'-(4,6-Dimethyl-pyrimidin-2-yl)-N"-ethoxy-N"'-(2-ethoxycarbonyl-benzolsulfonyl)-guanidin und 4-Fluor-benzolsulfonsäurechlorid als Ausgangsstoffe, so kann der Reaktionsablauf durch folgendes Formelschema skizziert werden:

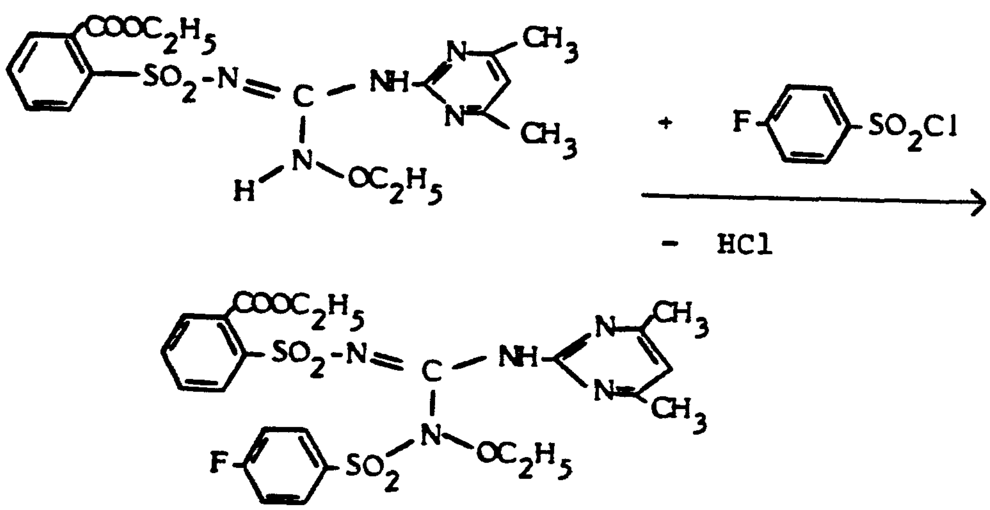

Verwendet man beispielsweise für die Verfahrensvariante (b) N'-(4,6-Dimethyl-pyrimidin-2-yl)-N"-allyloxy-N"-(2-brom-benzolsulfonyl)-guanidin und Methan-sulfonsäurechlorid als Ausgangsstoffe, so kann der Reaktionsablauf durch folgendes Formelschema skizziert werden:

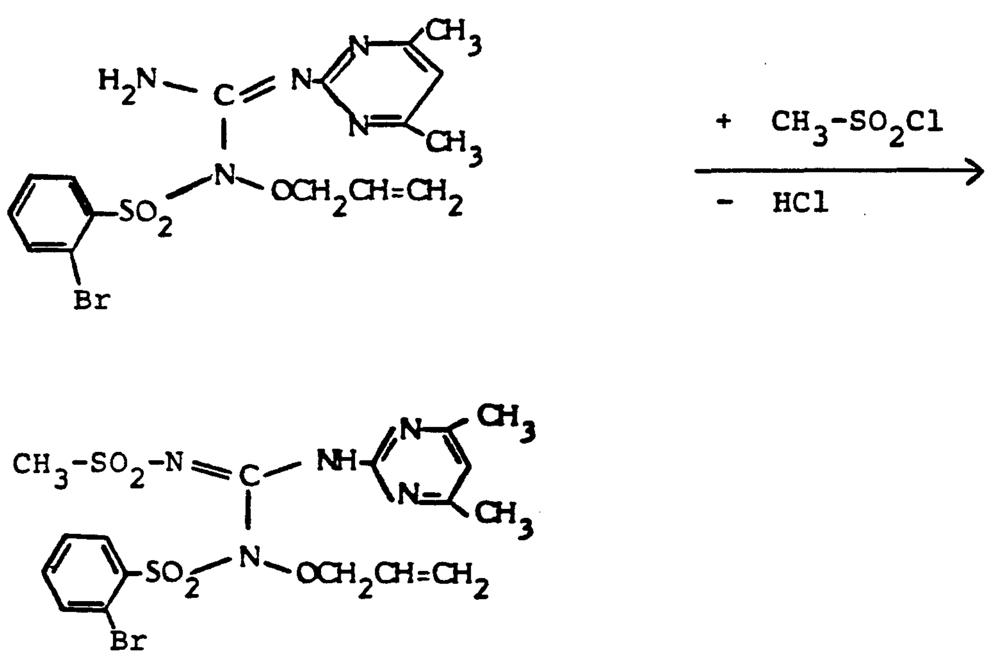


Verwendet man beispielsweise für die Verfahrensvariante (c) N'-(4,6-Dimethyl-pyrimidin-2-yl)-N"-phenoxy-N"-(2-chlor-benzolsulfonyl)-N"'-(3-chlor-benzol-sulfonyl)-guanidin und Kalium-ethanolat als Ausgangsstoffe, so kann der Reaktionsablauf durch folgendes Reaktionsschema skizziert werden:

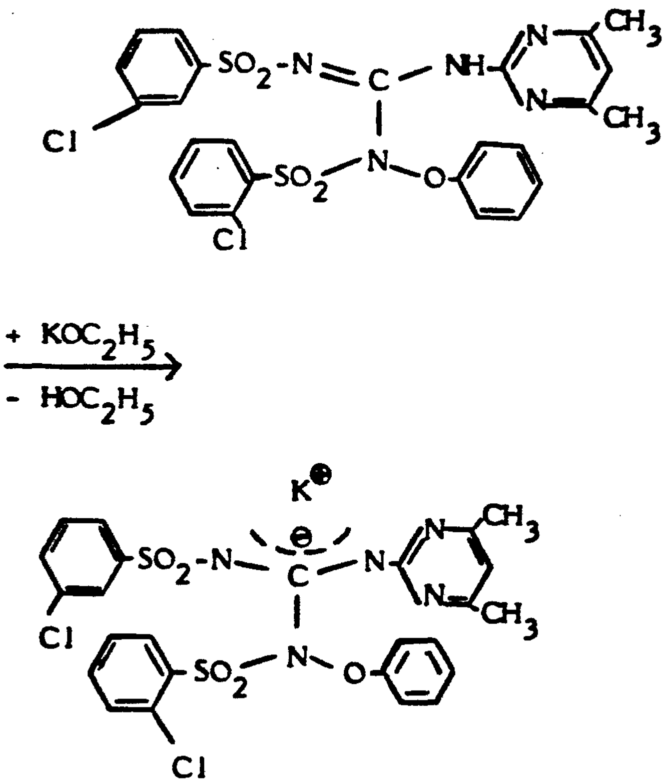


Verwendet man beispielsweise für die Verfahrensvariante (d) N'-(4,6-Dimethyl-pyrimidin-2-yl)-N"-benzyloxy-N"-(2-chlor-benzolsulfonyl)-N"'-(2-methylthiomethyl-benzolsulfonyl)-guanidin und Trifluormethansulfonsäure als Ausgangsstoffe, so kann der Reaktionsablauf durch folgendes Formelschema skizziert werden:

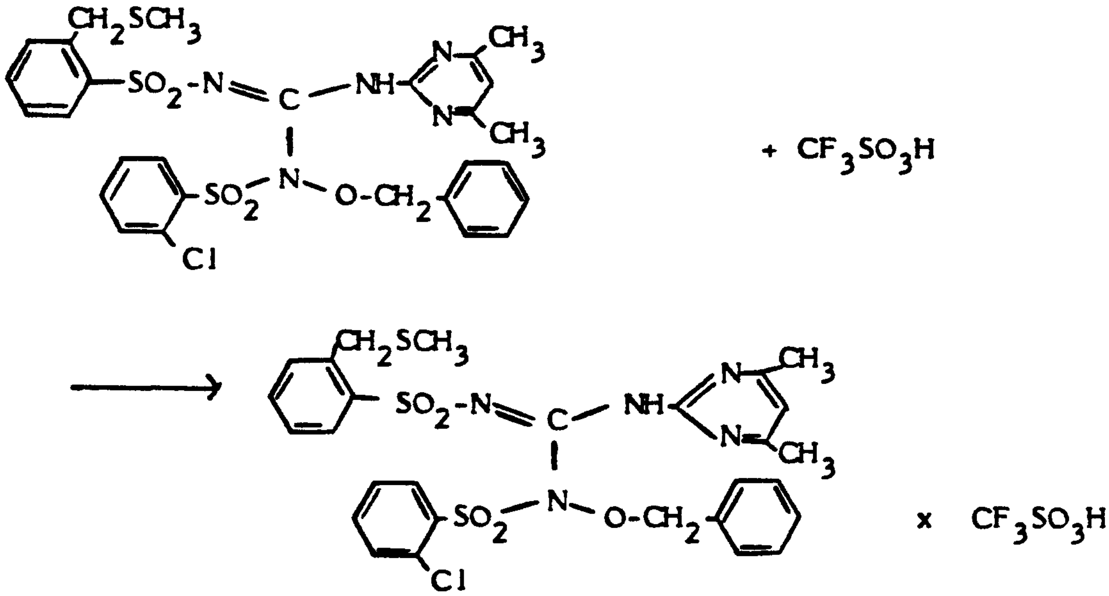


Die als Ausgangsstoffe für die Verfahrensvariante (a) zu verwendenden Sulfonylguanidine sind durch die Formel (II) allgemein definiert. In Formel (II) haben $A^1$ und R vorzugsweise die gleichen Bedeutungen, wie sie oben im Rahmen der Substituentendefinition der Formel (I) vorzugsweise angegeben sind.

Als Ausgangsstoffe der Formel (II) seien beispielsweise genannt:
N'-(4,6-Dimethyl-pyrimidin-2-yl)-N"-methoxy-N"'-benzolsulfonyl-, -N"'-(2-chlor-benzolsulfonyl)-, -N"'-(3-chlor-

benzolsulfonyl)-, -N"'-(4-chlor-benzolsulfonyl)-, N"'-(2-fluor-benzolsulfonyl)-, -N"'-(4-fluor-benzolsulfonyl)-, -N"'-(2-cyclopropyloxy-carbonyl-benzolsulfonyl)-, -N"'-(2-trifluormethylthio-benzolsulfonyl)-, -N"'-(2-difluormethylthiomethyl-benzolsulfonyl)-, -N"'-(2-N-methoxy-N-methylaminosulfonyl-benzolsulfonyl)-, -N"'-(2-brom-benzolsulfonyl)-, -N"'-(4-brom-benzolsulfonyl)-, -N"'-(2-cyano-benzolsulfonyl)-, -N"'-(2-nitro-benzolsulfonyl)-, -N"'-(4-nitro-benzolsulfonyl)-, -N"'-(2-methyl-benzolsulfonyl)-, -N"'-(4-methyl-benzolsulfonyl)-, -N"'-(2-chlormethyl-benzolsulfonyl)-, -N"'-(2-trifluormethyl-benzolsulfonyl)-, -N"'-(2-methoxy-benzolsulfonyl)-, -N"'-(4-methoxy-benzolsulfonyl)-, -N"'-(2-methylthio-benzolsulfonyl)-, -N"'-(2-difluormethoxy-benzolsulfonyl)-, -N"'-(2-trifluormethoxy-benzolsulfonyl)-, -N"'-(2-methylthio-methyl-benzolsulfonyl)-, -N"'-(2-dimethylamino-sulfonyl-benzolsulfonyl)-, -N"'-(2-phenyl-benzolsulfonyl)-, -N"'-(2-methoxysulfonyl-benzolsulfonyl)-, -N"'-(2-methoxycarbonyl-benzolsulfonyl)-, -N"'-(2-ethoxycarbonyl-benzolsulfonyl)-, -N"'-(2-propoxycarbonyl-benzolsulfonyl)-, -N"'-(2-methylaminocarbonyl-benzolsulfonyl)-, -N"'-(2-ethylaminocarbonyl-benzolsulfonyl)-, -N"'-(2-propylaminocarbonyl-benzolsulfonyl)-, -N"'-(2-methoxyaminocarbonyl-benzolsulfonyl)-, -N"'-(2-ethoxyaminocarbonyl-benzolsulfonyl)-, -N"'-(2-propoxyaminocarbonyl-benzolsulfonyl)-, -N"'-(2-dimethylaminocarbonyl-benzolsulfonyl)- und -N"'-(2-diethylaminocarbonyl-benzolsulfonyl)-guanidin; ferner N'-(4,6-Dimethyl-pyrimidin-2-yl)-N"-ethoxy-N"'-benzolsulfonyl-, -N"'-(2-chlor-benzol-sulfonyl)-, -N"'-(3-chlor-benzolsulfonyl)-, -N"'-(4-chlor-benzolsulfonyl)-, -N"'-(2,4-dichlor-benzolsulfonyl)-, -N"'-(2,5-dichlor-benzolsulfonyl), -N"'-(2-difluormethoxy-benzolsulfonyl)-, -N"'-(2-trifluormethoxy-benzolsulfonyl)-, -N"'-(2-methylthio-benzolsulfonyl)-, -N"'-(2-methyl-benzolsulfonyl)-, -N"'-(2-methylthiomethyl-benzolsulfonyl)-, -N"'-(2-dimethyl-aminosulfonyl-benzolsulfonyl)-, -N"'-(2-methoxysulfonyl-benzolsulfonyl)-, -N"'-(2-methoxycarbonyl-benzolsulfonyl)-, -N"'-(2-ethoxycarbonyl-benzolsulfonyl)-, -N"'-(2-methylaminocarbonyl-benzolsulfonyl)-, -N"'-(2-ethylaminocarbonyl-benzolsulfonyl)-, -N"'-(2-methoxyaminocarbonyl-benzolsulfonyl)- und -N"'-(2-dimethylaminocarbonxyl-benzolsulfonyl)-guanidin; ferner

N'-(4,6-Dimethyl-pyrimidin-2-yl)-N"-propoxy-, -N"-isopropoxy-, -N"-butoxy-, -N"-isobutoxy-, -N"-sec.-butoxy-, -N"-pentoxy-, -N"-hexyloxy-, -N"-octyloxy-, -N"-allyloxy-, -N"-crotyloxy-, -N"-(3-chlor-propoxy)-, -N"-methoxycarbonyl-methoxy-, -N"-ethoxycarbonylmethoxy-, -N"-(1-methoxycarbonyl-ethoxy)-, -N"-(1-ethoxycarbonyl-ethoxy)-, -N"-(2-phenyl-ethoxy)-, -N"-phenoxy-, -N"-benzyloxy-, -N"-(4-methyl-benzyloxy)-, -N"-(4-fluor-benzyloxy)-, -N"-(4-chlor-benzyloxy)-, -N"-(4-nitro-benzyloxy)-, -N"-(2,6-dichlor-benzyloxy)-, -N"-(4-methoxycarbonyl-benzyloxy)- und -N"-(4-ethoxycarbonyl-benzyloxy)-, -N"'-(2-chlor-benzolsulfonyl)-guanidin und -N"'-(2-methoxycarbonyl-benzolsulfonyl)-guanidin sowie

N'-(4,6-Dimethyl-pyrimidin-2-yl)-N"-methoxy-, -N"-ethoxy- und -N"-benzyloxy-, -N"'-methylsulfonyl-, -N"'-ethylsulfonyl-, -N"'-trifluormethylsulfonyl-, -N"'-(2-chlor-ethylsulfonyl)-, -N"'-methylaminosulfonyl-, -N"'-isopropylaminosulfonyl-, -N"'-dimethylaminosulfonyl-, -N"'-perfluoroctylaminosulfonyl- und -N"'-methyl-trifluormethyl-aminosulfonyl-guanidin.

Die Ausgangsstoffe der Formel (II) sind bekannt (vgl. DE-OS 3 334 455).

Man erhält die Verbindungen der Formel (II), wenn man

($a^1$) Verbindungen der Formel (VI)

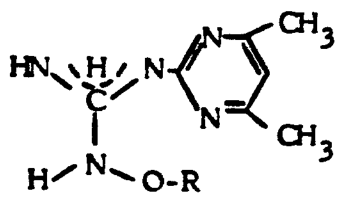

(VI)

in welcher

R die oben angegebene Bedeutung hat,

mit Sulfonsäurechloriden der Formel (V)

$$A^1\text{-}SO_2\text{-}Cl \quad (V)$$

in welcher

$A^1$ die oben angegebene Bedeutung hat,

in Gegenwart von Säureakzeptoren, wie z.B. Pyridin oder Diazabicyclooctan (DABCO) und gegebenenfalls in Gegenwart von Verdünnungsmitteln, wie z.B. Methylenchlorid oder Chloroform, bei Temperaturen zwischen -20°C und +30°C umsetzt;

oder

(a²) Sulfonylguanidine der Formel (VII)

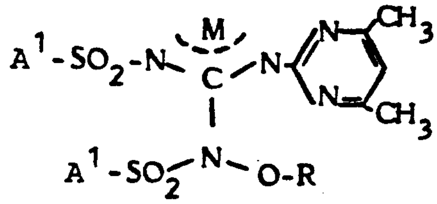

(VII)

in welcher

$A^1$, R und M die oben angegebenen Bedeutungen haben,

mit Hydroxylamin-Derivaten der Formel (VIII)

$H_2N-O-R$ (VIII)

in welcher

R die oben angegebene Bedeutung hat,

oder mit Hydrochloriden von Verbindungen der Formel (VIII),

gegebenenfalls in Gegenwart von Verdünnungsmitteln, wie z.B. Ethanol, bei Temperaturen zwischen 20°C und 100°C umsetzt;

oder

(a3) Verbindungen der Formel (IX)

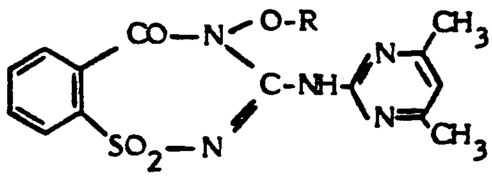

(IX)

in welcher

R die oben angegebene Bedeutung hat,

mit Natrium- oder Kalium-alkanolaten mit bis zu 6 Kohlenstoffatomen bzw.

mit Aminoverbindungen der Formel (X)

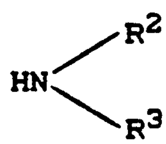

(X)

in welcher

$R^2$ für Wasserstoff oder $C_1$-$C_4$-Alkyl steht und

$R^3$ für $C_1$-$C_4$-Alkyl, $C_1$-$C_4$-Alkoxy oder Dimethylamino steht,

gegebenenfalls in Gegenwart von Verdünnungsmitteln, wie z.B. Methanol oder Ethanol, bei Temperaturen zwischen 0°C und 100°C umsetzt;

oder

($a^4$) Verbindungen der Formel (XI)

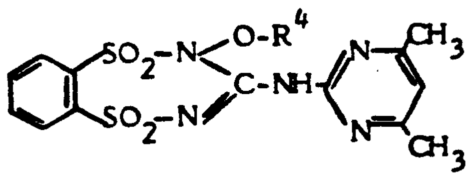

(XI)

in welcher

$R^4$ für $C_1$-$C_4$-Alkyl steht,

mit Hydroxylamin-Derivaten der Formel (VIII)

$H_2N$-O-R (VIII)

in welcher

R die oben angegebene Bedeutung hat,

gegebenenfalls in Gegenwart von Verdünnungsmitteln, wie z.B. Methylenchlorid, bei Temperaturen zwischen 0°C und 100°C umsetzt.

Die oben unter ($a^1$) angegebenen Guanidin-Zwischenprodukte sind durch die Formel (VI) allgemein definiert. In Formel (VI) hat R vorzugsweise die gleiche Bedeutung, wie sie oben im Rahmen der Substituentendefinition der Formel (I) vorzugsweise angegeben ist.

Als Ausgangsstoffe der Formel (VI) seien beispielsweise genannt:
N'-(4,6-Dimethyl-pyrimidin-2-yl)-N"-methoxy-guanidin, -N"-ethoxy-guanidin, -N"-propoxy-guanidin, -N"-isopropoxy-guanidin, -N"-butoxy-guanidin, -N"-isobutoxy-guanidin, -N"-sec-butoxy-guanidin, -N"-pentoxy-guanidin, -N"-iso-pentoxy-guanidin, -N"-hexyloxy-guanidin, -N"-isohexyloxy-guanidin, -N"-heptyloxy-guanidin, -N"-isoheptyloxy-guanidin, -N"-octyloxy-guanidin, -N"-isooctyloxy-guanidin, -N"-allyloxy-guanidin, -N"-crotyloxy-guanidin, -N"-(2-chlor-ethoxy)-guanidin, -N"-(2-fluor-ethoxy)-guanidin, -N"-(2-chlor-propoxy)-guanidin, -N"-(3-chlor-propoxy)-guanidin, -N"-(4-chlor-butoxy)-guanidin, -N"-methoxycarbonylmethoxy-guanidin, -N"-ethoxycarbonyl-methoxy-guanidin, -N"-(1-methoxycarbonyl-ethoxy)-guanidin, -N"-(1-ethoxycarbonyl-ethoxy)-guanidin, -N"-aminocarbonyl-methoxy-guanidin, -N"-(2-phenyl-ethoxy)-guanidin, -N"-phenoxy-guanidin, -N"-(4-methyl-benzyloxy)-guanidin, -N"-(4-fluor-benzyloxy)-guanidin, -N"-(4-chlor-benzyloxy)-guanidin, -N"-(4-nitro-benzyloxy)-guanidin, -N"-(2,6-dichlor-benzyloxy)-guanidin, -N"-(4-ethoxycarbonyl-benzyloxy)-guanidin und -N"-(4-methoxycarbonyl-benzyloxy)-guanidin.

Die Ausgangsstoffe der Formel (VI) sind zum Teil bekannt (vgl. J. Chem. Soc. 1962, S. 3915 und DE-OS 3 334 445).

Man erhält die Verbindungen der Formel (VI), wenn man 2-Cyanamino-4,6-dimethyl-pyrimidin der Formel (XII)

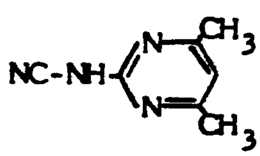

(XII)

mit Hydroxylamin-Derivaten der Formel (VIII)

$$H_2N\text{-}O\text{-}R \qquad (VIII)$$

in welcher

R die oben angegebene Bedeutung hat,

bzw. mit Hydrochloriden von Verbindungen der Formel (VIII), gegebenenfalls in Gegenwart von Verdünnungsmitteln, wie z.B. Ethanol, Isopropanol oder Butanol, bei Temperaturen zwischen 20°C und 150°C, vorzugsweise zwischen 50°C und 120°C, umsetzt und gegebenenfalls die Umsetzungsprodukte mit Säureakzeptoren, wie z.B. Ammoniak, Natronlauge oder Soda, behandelt.

2-Cyanamino-4,6-dimethyl-pyrimidin der Formel (XII) ist bereits bekannt (vgl. J. Chem. Soc. 1953, 1725).

Hydroxylamin-Derivate der Formel (VIII) sind ebenfalls bereits bekannt und können nach an sich bekannten Verfahren hergestellt werden (vgl. Chem. Pharm. Bull. 15 (1967), 345; Bull. Soc. Chim. France 1958, 664; Synthesis 1976, 682: J. Chem. Soc. 1930, 228 und Helv. Chim. Acta 45 (1962), 1387).

Die oben unter ($a^1$) weiter als Ausgangsstoffe angegebenen Sulfonsäurechloride sind durch die Formel (V) allgemein definiert. In der Formel (V) hat $A^1$ vorzugsweise die gleiche Bedeutung, wie sie oben im Rahmen der Substituentendefinition der Formel (I) vorzugsweise angegeben ist.

Als Ausgangsstoffe der Formel (V) seien beispielsweise genannt:
Benzolsulfonsäurechlorid, 2-Chlor-, 3-Chlor-, 4-Chlor-, 2,5-Dichlor-, 2-Fluor-, 4-Fluor-, 2-Brom-, 4-Brom-, 2-Cyano-, 2-Nitro-, 4-Nitro-, 2-Methyl-, 4-Methyl-, 2-Chlormethyl-, 2-Trifluormethyl-, 2-Methoxy-, 4-Methoxy-, 2-Methylthio-, 2-Trifluormethylthio-, 2-Difluormethylthio-, 2-Cyclopropyloxycarbonyl-, 2-Phenoxy-, 2-Difluormethoxy-, 2-Trifluormethoxy-, 2-Methylthiomethyl-, 2-Dimethylaminosulfonyl-, 2-Phenyl-, 2-Methoxycarbonyl-, 2-Ethoxycarbonyl-, 2-Dimethylaminocarbonyl- und 2-Diethylaminocarbonylbenzolsulfonsäurechlorid sowie (2-Chlor-phenyl)-, (2-Cyano-phenyl)- und (2-Methoxycarbonyl-phenyl)-methansulfonsäurechlorid, ferner Methan-, Chlormethan-, Fluormethan-, Difluormethan-, Trifluormethan-, Ethan-, 2,2,2-Trifluorethan, 2,2,2-Trichlorethan, 2,2-Difluorethan-, 1-Fluor-ethan, 2-Fluorethan-, 2-Chlorethan-, 2-Bromethan-, Ethen-, Perfluorethen-, Perchlorethen-, 2,2-Difluorethen-, 2,2-Dichlorethen-, Propan-, 3-Chlor-propan-, 3-Fluorpropan-, 2-Fluorpropan-, Chlorfluormethan-, Dichlorfluormethan-, Difluorchlormethan-, Butan-, 4-Chlor-butan-, Perfluorbutan-, 1-Methylethan-, Pentan-, Hexan-, Heptan-, Octan-, Perfluoroctan-, Cyclopropan-, Cyclobutan-, Cyclopentan-, Cyclohexan-sulfonsäurechlorid;
ferner Methyl-, Ethyl-, Propyl-, Isopropyl-, Butyl-, Isobutyl-, sec.-Butyl-, Hexyl-, 2,4-Dimethylbutyl-, 2-Chlor-1-ethyl-ethyl-, 2-Methoxy-1-methyl-ethyl-, 2-Chlorethyl-, 1-Fluorethyl-, 2-Chlor-1-ethyl-propyl-, 2-Chlor-2-methylpropyl-, 2-Chlor-1,2-dimethyl-propyl-, 1,1-Dimethylethyl-, 1,1-Dimethylpropyl-, Methoxy-, Ethoxy-, Propoxy-, Isopropoxy-, N-Chlormethyl-N-methyl-, N-Chlormethyl-N-ethyl-,

N-Chlormethyl-N-isopropyl-, N-Ethenyl-N-methyl-, N-Methoxymethyl-N-methyl-, N-Allyloxymethyl-N-methyl-, N-Methoxycarbonylmethyl-N-methyl-, N,N-Dimethyl-, N,N-Diethyl- und N,N-Dipropyl-sulfamidsäurechlorid.

Die Sulfonsäurechloride der Formel (V) sind bekannt und können nach an sich bekannten Verfahren hergestellt werden (vgl. J. Org. Chem. 33 (1968), 2104; J. Org. Chem. 25 (1960), 1824; DE-AS 2 308 262; EP-OS 23 140, 23 141, 23 422, 35 893, 48 143, 51 466, 64 322, 70 041, 44 808 und 44 809; US-PS 2 929 820, 4 282 242; 4 348 220 und 4 372 778 sowie Angew. Chem. 93 (1981), 151).

Die oben unter ($a^2$) angegebenen Sulfonylguanidine sind durch die Formel (VII) allgemein definiert. In Formel (VII) haben $A^1$, R und M vorzugsweise die gleichen Bedeutungen, wie sie oben im Rahmen der Substituentendefinition der Formel (I) vorzugsweise angegeben sind.

Als Ausgangsstoffe der Formel (VII) seien beispielsweise genannt:
N'-(4,6-Dimethyl-pyrimidin-2-yl)-N"-methoxy-N",N"'-bis-(2-chlor-benzolsulfonyl)-guanidin, -N",N"'-bis-(2-brom-benzolsulfonyl)-guanidin, -N",N"'-bis-(2-fluor-benzolsulfonyl)-guanidin, -N",N"'-bis-(2-methyl-benzolsulfonyl)-guanidin, -N",N"'-bis-(2-trifluormethyl-benzolsulfonyl)-guanidin, -N",N"'-bis-(2-methoxy-benzolsulfonyl-guanidin, N",N"'-bis-(2-phenyl-benzolsulfonyl)-guanidin und -N",N"'-bis-(2-methoxycarbonyl-benzolsulfonyl)-guanidin.

Die Verbindungen der Formel (VII) sind bekannt (vgl. DE-OS 3 334 455).

Man erhält die Sulfonylguanidine der Formel (VII) analog dem oben unter (a[1]) beschriebenen Verfahren, wobei jedoch wenigstens 2 Moläquivalente der Säurechloride der Formel (V) und wenigstens 2 Moläquivalente der Säureakzeptoren einzusetzen sind.

Die oben unter (a[2]) weiter als Ausgangsstoffe angegebenen Hydroxylamine sind durch die Formel (VIII) allgemein definiert.

In Formel (VIII) hat R vorzugsweise die gleiche Bedeutung, wie sie oben im Rahmen der Substituentendefinition der Formel (I) vorzugsweise angegeben ist.

Als Ausgangsstoffe der Formel (VIII) seien beispielsweise genannt:
O-Propyl-, O-Isopropyl-, O-Butyl-, O-Isobutyl-, O-sec.-Butyl-, O-Pentyl-, O-Isopentyl-, O-sec.-Pentyl-, O-Hexyl-, O-Isohexyl-, O-Heptyl-, O-Isoheptyl-, O-Octyl-, O-Isooctyl-, O-Allyl-, O-Crotyl-, O-(2-Chlor-ethyl)-, O-(2-Fluor-ethyl)-, O-(2-Chlor-propyl)-, O-(3-Chlor-propyl)-, O-(4-Chlor-butyl)-, O-Methoxycarbonylmethyl-, O-Ethoxycarbonylmethyl-, O-(1-Methoxycarbonyl)-ethyl-, O-(1-Ethoxycarbonyl)-ethyl-, O-Aminocarbonylmethyl-, O-(2-Phenyl-ethyl)-, O-Phenyl-, O-(4-Methyl-benzyl)-, O-(4-Fluor-benzyl)-, O-(4-Chlor-benzyl)-, O-(4-Nitro-benzyl)-, O-(2,6-Dichlorbenzyl)-, O-(4-Methoxycarbonyl-benzyl)- und O-(4-Ethoxy-carbonyl-benzyl)-hydroxylamin.

Hydroxylamin-Derivate der Formel (VIII) sind bekannt und können nach an sich bekannten Verfahren hergestellt werden

(vgl. Chem. Pharm. Bull. 15 (1967), 345; Bull. Soc. Chim. France 1958, 664; Synthesis 1976, 682; J. Chem. Soc. 1930, 222 and Helv. Chim. Acta 45 (1962), 1387).

Die oben unter ($a^3$) als Ausgangsstoffe angegebenen cyclischen Zwischenprodukte sind durch die Formel (IX) allgemein definiert. In Formel (IX) hat R vorzugsweise die gleiche Bedeutung, wie sie oben im Rahmen der Substituentendefinition der Formel (I) vorzugsweise angegeben ist.

Als Beispiele seien Verbindungen der Formel (IX) genannt, in welcher R für Methyl, Ethyl, Propyl, Isopropyl, Butyl, iso-Butyl, sec.-Butyl, Octyl, Benzyl, Allyl, Crotyl, 3-Chlor-propyl, Methoxycarbonylmethyl, Ethoxycarbonylmethyl, 1-Methoxycarbonyl-ethyl, 1-Ethoxycarbonyl-ethyl, Aminocarbonylmethyl, 2-Phenyl-ethyl, 4-Methyl-ethyl, 4-Fluorbenzyl, 4-Chlor-benzyl, 4-Nitro-benzyl oder 4-Methoxycarbonyl-benzyl steht.

Die Verbindungen der Formel (IX) sind noch nicht in der Literatur beschrieben. Man erhält die Verbindungen der Formel (IX), wenn man Guanidin-Derivate der Formel (VI)

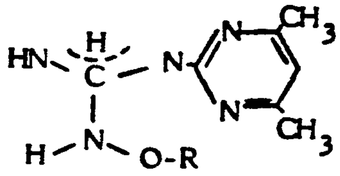

(VI)

in welcher

R die oben angegebene Bedeutung hat,

mit 2-Chlorsulfonyl-benzoylchlorid der Formel (XIII)

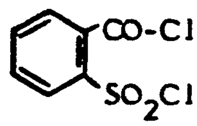

(XIII)

gegebenenfalls in Gegenwart von Säureakzeptoren, wie z.B. Pyridin oder Diazabicyclooctan (DABCO), und gegebenenfalls in Gegenwart von Verdünnungsmitteln, wie z.B. Methylenchlorid oder Chloroform, bei Temperaturen zwischen -20°C und +30°C umsetzt.

Die Herstellung der Zwischenprodukte der Formel (VI) wurde bereits weiter oben im Zusammenhang mit dem unter ($a^1$) angegebenen Verfahren beschrieben.

2-Chlorsulfonyl-benzoylchlorid der Formel (XIII) ist bekannt (vgl. DE-OS 2 036 171).

Die oben unter ($a^4$) als Ausgangsstoffe angegebenen cyclischen Zwischenprodukte sind durch die Formel (XI) allgemein definiert. In Formel (XI) steht $R^4$ vorzugsweise für $C_1$-$C_3$-Alkyl.

Als Beispiele seien Verbindungen der Formel (XI) genannt, in welcher $R^4$ für Methyl, Ethyl, Propyl oder Isopropyl steht.

Die Verbindungen der Formel (XI) sind noch nicht in der Literatur beschrieben. Man erhält die Verbindungen der Formel (XI), wenn man Guanidin-Derivate der Formel (XIV)

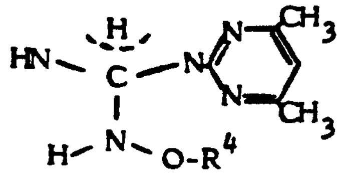

(XIV)

in welcher

$R^4$ die oben angegebene Bedeutung hat,

mit Benzol-1,2-disulfonsäure-dichlorid der Formel (XV)

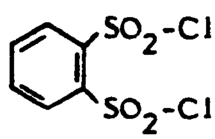

(XV)

gegebenenfalls in Gegenwart von Säureakzeptoren, wie z.B. Pyridin oder Diazabicyclooctan (DABCO), und gegebenenfalls in Gegenwart von Verdünnungsmitteln, wie z.B. Methylenchlorid oder Chloroform, bei Temperaturen zwischen -20°C und +30°C umsetzt.

Die Guanidin-Derivate der Formel (XIV) fallen unter die allgemeine Formel (VI). Die Herstellung der Verbindungen der Formel (VI) wurde bereits weiter oben im Zusammenhang mit dem unter ($a^1$) angegebenen Verfahren beschrieben.

Benzol-1,2-disulfonsäure-dichlorid der Formel (XV) ist bekannt (vgl. J. Org. Chem. 31 (1966), 3289-3292).

Die oben unter ($a^4$) weiter als Ausgangsstoffe angegebenen Hydroxylamin-Derivate sind ebenfalls bekannt und/oder können nach an sich bekannten Verfahren hergestellt werden (vgl. Chem. Pharm. Bull. 15 (1967), 345; Bull. Soc. Chim. France 1958, 664; Synthesis 1976, 682; J. Chem. Soc. 1930, 228 und Helv. Chim. Acta 45 (1962), 1387).

Die weiter als Ausgangsstoffe für das oben unter (a) angegebene erfindungsgemäße Herstellungsverfahren zu verwendenden Sulfonsäurechloride sind durch die Formel (III) allgemein definiert. In Formel (III) hat $A^2$ vorzugsweise die gleiche Bedeutung, wie sie oben im Rahmen der Substituentendefinition der Formel (I) vorzugsweise angegeben ist.

Als Beispiele für Verbindungen der Formel (III) können die gleichen Sulfonsäurechloride dienen, die oben im Zusammenhang mit der Beschreibung des unter ($a^1$) angegebenen Verfahrens als Beispiele für Verbindungen der Formel (V) genannt wurden.

Die Sulfonsäurechloride der Formel (III) sind bekannt und können nach an sich bekannten Verfahren hergestellt werden (vgl. Literaturangaben für die Ausgangsstoffe der Formel (V) zu Verfahren ($a^1$) oben).

Die als Ausgangsstoffe für die Verfahrensvariante (b) zu verwendenden Sulfonylguanidine sind durch die Formel (IV) allgemein definiert. In Formel (IV) haben $A^2$ und R vorzugsweise die gleichen Bedeutungen, wie sie oben im Rahmen der Substituentendefinition der Formel (I) vorzugsweise angegeben sind.

Als Ausgangsstoffe der Formel (IV) seien beispielsweise genannt:

N'-(4,6-Dimethyl-pyrimidin-2-yl)-N"-methoxy-N"-benzolsulfonyl-, -N"-(2-chlor-benzolsulfonyl)-, -N"-(3-chlor-benzolsulfonyl)-, -N"-(4-chlor-benzolsulfonyl)-, -N"-(2-fluor-benzolsulfonyl)-, -N"-(4-fluor-benzolsulfonyl)-, -N"-(2-brom-benzolsulfonyl)-, -N"-(2-difluormethylthio-benzolsulfonyl)-,-N"-(2-cyano-benzolsulfonyl)-, -N"-(2-nitro-benzolsulfonyl)-, -N"-(4-nitro-benzolsulfonyl)-, -N"-(2-methyl-benzolsulfonyl)-, -N"-(4-methyl-benzolsulfonyl)-, -N"'-(2-chlormethyl-benzolsulfonyl)-, -N"-(2-trifluormethyl-benzolsulfonyl)-, -N"-(2-methoxy-benzolsulfonyl)-, -N"-(4-methoxy-benzolsulfonyl)-, -N"-(2-methylthio-benzolsulfonyl)-, -N"-(2-difluormethoxy-benzolsulfonyl)-, -N"-(2-trifluormethoxy-benzolsulfonyl)-, -N"-(2-methylthiomethyl-benzolsulfonyl)-, -N"-(2-dimethylamino-sulfonyl-benzolsulfonyl)-, -N"-(2-phenyl-benzolsulfonyl)-, -N"-(2-methoxysulfonyl-benzolsulfonyl)-, -N"-(2-methoxycarbonyl-benzolsulfonyl)-, -N"-(2-ethoxycarbonyl-benzolsulfonyl)-, -N"-(2-propoxycarbonyl-benzolsulfonyl)-, -N"-(2-methyl-methoxy-amino-carbonyl-benzolsulfonyl)-, -N"-(2-dimethylaminocarbonyl-benzolsulfonyl)-, -N"-(2-diethylaminocarbonyl-benzolsulfonyl)-, -N"-(2-trifluormethylthio-benzolsulfonyl)-, -N"-(2-cyclopropyloxycarbonyl-benzolsulfonyl)- und -N"-(2-phenoxy-benzolsulfonyl)-guanidin;

ferner N'-(4,6-Dimethyl-pyrimidin-2-yl)-N"-ethoxy-N"-benzolsulfonyl-, -N"-(2-chlor-benzol-sulfonyl)-, -N"-(3-chlor-benzolsulfonyl)-, -N"-(4-chlor-benzolsulfonyl)-, -N"-(2,4-dichlor-benzolsulfonyl)-, -N"-(2,5-dichlor-ben-

zolsulfonyl)-, -N"(4-nitro-benzolsulfonyl)-, -N"-(4-methoxy-benzolsulfonyl)-, -N"-(4-fluor-benzolsulfonyl)- und -N"-(4-brom-benzolsulfonyl)-guanidin; ferner N'-(4,6-Dimethyl-pyrimidin-2-yl)-N"-propoxy-, -N"-isopropoxy-, -N"-butoxy-, -N"-isobutoxy-, -N"-sec.-butoxy-, -N"-pentoxy-, -N"-allyloxy-, -N"-crotyloxy-, -N"-(3-chlor-propoxy)-, -N"-methoxycarbonylmethoxy-, -N"-ethoxycarbonylmethoxy-, -N"-(2-phenyl-ethoxy)-, -N"-benzyloxy-, -N"-(4-methylbenzyloxy)-, -N"-(4-fluor-benzyloxy)-, -N"-(4-chlor-benzyloxy)-, -N"-(4-nitro-benzyloxy)-, -N"-(2,6-dichlor-benzyloxy)-, -N"-(4-methoxycarbonyl-benzyloxy)- und -N"-(4-ethoxycarbonyl-benzyloxy)-, -N"-(2-chlorbenzolsulfonyl)-guanidin sowie -N"-(2-methoxycarbonylbenzolsulfonyl)-guanidin.

Die Ausgangsstoffe der Formel (IV) sind bekannt (vgl. DE-OS 3 334 455).

Man erhält die Verbindungen der Formel (IV), wenn man Guanidin-Derivate der Formel (VI)

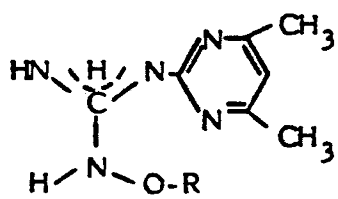

(VI)

in welcher

R die oben angegebene Bedeutung hat,

mit Sulfonsäurechloriden der Formel (III)

$$A^2\text{-}SO_2\text{-}Cl \qquad (III)$$

in welcher

$A^2$ die oben angegebene Bedeutung hat,

in Gegenwart von Säureakzeptoren, wie z.B. Triethylamin, Pyridin oder Diazabicyclooctan (DABCO) und gegebenenfalls in Gegenwart von Verdünnungsmitteln, wie z.B. Methylenchlorid, Chloroform oder Tetrahydrofuran bei Temperaturen zwischen -20°C und +30°C umsetzt.

Die Herstellung der Zwischenprodukte der Formel (VI) wurde bereits weiter oben im Zusammenhang mit dem unter ($a^1$) angegebenen Verfahren beschrieben.

Die Sulfonsäurechloride der Formel (III) sind bekannt und können nach an sich bekannten Verfahren hergestellt werden (vgl. Literaturangaben für die Ausgangsstoffe der Formel (V) zu Verfahren ($a^1$) oben).

Die weiter als Ausgangsstoffe für das oben unter (b) angegebene erfindungsgemäße Herstellungsverfahren zu verwendenden Sulfonsäurechloride sind durch die Formel (V) allgemein definiert. In Formel (V) hat $A^1$ vorzugsweise die gleiche Bedeutung, wie sie oben im Rahmen der Substituentendefinition der Formel (I) vorzugsweise angegeben ist.

Beispiele für Verbindungen der Formel (V) und Literaturangaben zu deren Herstellung sind der Beschreibung des oben unter ($a^1$) angegebenen Verfahrens zu entnehmen.

Die bei der Verfahrensvariante (c) als Ausgangsstoffe zu verwendenden Sulfonylguanidine sind durch die Formel (I) - mit der Maßgabe, daß M für Wasserstoff steht - allgemein definiert. In Formel (I) - soweit sie die als Ausgangsstoffe für Verfahren (c) zu verwendenden Verbindungen betrifft - steht M für Wasserstoff, und $A^1$, $A^2$ und R haben vorzugsweise die gleichen Bedeutungen, wie sie oben im Rahmen der Substituentendefinition für Formel (I) vorzugsweise angegeben sind.

Die als Ausgangsstoffe für Verfahren (c) zu verwendenden Verbindungen der Formel (I) können nach den unter (a) und (b) beschriebenen Verfahren hergestellt werden.

Als Beispiele für die bei Verfahren (c) zu verwendenden Metallhydroxide, -hydride, -alkanolate bzw. metallorganischen Verbindungen seien genannt:
Lithium-, Natrium-, Kalium-, Magnesium- und Calcium-hydroxid, Lithium-, Natrium- und Calcium-hydrid, Natriummethanolat und -ethanolat, Kalium-methanolat, -ethanolat und Kalium-tert.-butanolat sowie Butyllithium und Isopropylmagnesiumchlorid.

Die bei der Verfahrensvariante (d) als Ausgangsstoffe zu verwendenden Sulfonylguanidine sind durch die Formel (I) - mit der Maßgabe, daß M für Wasserstoff steht - allgemein definiert. In Formel (I) - soweit sie die als Ausgangsstoffe für Verfahren (d) zu verwendenden Verbindungen betrifft - steht M für Wasserstoff und $A^1$, $A^2$ und R haben vorzugsweise die gleichen Bedeutungen, wie sie oben im Rahmen der Substituentendefinition der Formel (I) vorzugsweise angegeben sind.

Die als Ausgangsstoffe für Verfahren (d) zu verwendenden Verbindungen der Formel (I) können nach den unter (a) und (b) beschriebenen Verfahren hergestellt werden.

Bei Verfahren (d) werden starke Säuren als Ausgangsstoffe eingesetzt. Vorzugsweise sind dies Halogenwasserstoffsäuren, wie Hydrogenchlorid, Hydrogenbromid und Hydrogeniodid, weiter Schwefelsäure oder gegebenenfalls durch Fluor oder Chlor substituierte Alkansulfonsäuren mit bis zu 4 Kohlenstoffatomen, wie z.B. Methansulfonsäure, Ethansulfonsäure, Chlormethansulfonsäure, 2-Chlorethansulfonsäure und Trifluormethansulfonsäure, Trifluoressigsäure, ferner Benzolsulfonsäure, Naphthalin-1-sulfonsäure, Naphthalin-2-sulfonsäure, Naphthalin-1,4-, -1,5-, -1,6-, -2,6- und -2,7-disulfonsäure.

Das erfindungsgemäße Verfahren (a) zur Herstellung der neuen Verbindungen der Formel (I) wird vorzugsweise unter Verwendung von Verdünnungsmitteln durchgeführt. Als solche kommen praktisch alle inerten organischen, vorzugsweise jedoch aprotisch polare Solventien in Betracht. Hierzu gehören gegebenenfalls substituierte Kohlenwasserstoffe, wie z.B. Methylenchlorid, Chloroform, 1,2-Dichlorethan, Toluol, Xylol und Chlorbenzol, Nitrile, wie z.B. Acetonitril und Propionitril, Ether, wie z.B. 1,2-Dimethoxyethan, Tetrahydrofuran und Dioxan, sowie Dimethylformamid, Dimethylacetamid, Dimethylsulfoxid, Sulfolan, Pyridin und 2-Methyl-5-ethyl-pyridin.

Als Säureakzeptoren können bei Verfahren (a) praktisch alle üblicherweise verwendeten Säurebindemittel eingesetzt

werden. Hierzu gehören insbesondere Alkalimetall- und Erdalkalimetall-hydride, metallorganische Verbindungen, wie Butyllithium, ferner aliphatische, aromatische oder heterocyclische Amine, wie Trimethylamin, Triethylamin, N,N-Dimethylanilin, N,N-Dimethyl-benzylamin, Diazabicyclooctan (DABCO), Diazabicyclononen (DBN), Diazabicycloundecen (DBU), Pyridin, 2-Methyl-5-ethyl-pyridin und 4-Dimethylamino-pyridin.

Die Reaktionstemperaturen können bei Verfahren (a) innerhalb eines größeren Bereiches variiert werden. Im allgemeinen arbeitet man zwischen -80°C und +100°C, vorzugsweise zwischen -30°C und +50°C. Das erfindungsgemäße Verfahren (a) wird im allgemeinen bei Normaldruck durchgeführt.

Zur Durchführung von Verfahren (a) setzt man je Mol Sulfonylguanidin der Formel (II) im allgemeinen zwischen 1 und 2 Mol, vorzugsweise zwischen 1,1 und 1,5 Mol Sulfonsäurechlorid der Formel (III) ein.

Die Reaktionskomponenten werden gewöhnlich bei Raumtemperatur oder unter Außenkühlung zusammengegeben und das Reaktionsgemisch wird bis zum Reaktionsende gerührt.

Die Aufarbeitung und Isolierung der neuen Verbindungen erfolgt nach üblichen Methoden. Gegebenenfalls nach Abdestillieren flüchtiger Komponenten wird mit Wasser und einem mit Wasser praktisch nicht mischbaren Lösungsmittel, wie z.B. Methylenchlorid oder Chloroform geschüttelt, die

organische Phase mit Wasser gewaschen, getrocknet, filtriert und eingeengt. Die im Rückstand verbleibenden Produkte der Formel (I) werden durch Digerieren mit organischen Lösungsmitteln, wie z.B. Diethylether, Essigester, Ethanol oder Isopropanol zur Kristallisation gebracht und gegebenenfalls durch Umkristallisation gereinigt.

Das erfindungsgemäße Verfahren (b) zur Herstellung der neuen Verbindungen der Formel (I) wird vorzugsweise unter Verwendung von Verdünnungsmitteln durchgeführt. Als solche kommen praktisch alle inerten organischen, vorzugsweise jedoch aprotisch polare Solventien in Betracht, wie sie oben im Zusammenhang mit der Beschreibung des erfindungsgemäßen Verfahrens (a) genannt wurden.

Als Säureakzeptoren können bei Verfahren (b) praktisch alle üblicherweise verwendeten Säurebindemittel eingesetzt werden. Hierzu gehören insbesondere die oben im Zusammenhang mit der Beschreibung des erfindungsgemäßen Verfahrens (a) genannten Säurebindemittel.

Die Reaktionstemperaturen können bei Verfahren (b) innerhalb eines größeren Bereichs variiert werden. Im allgemeinen arbeitet man zwischen -80°C und +100°C, vorzugsweise zwischen -30°C und +50°C. Das Verfahren (b) wird im allgemeinen bei Normaldruck durchgeführt.

Zur Durchführung von Verfahren (b) setzt man je Mol Sulfonylguanidin der Formel (IV) im allgemeinen zwischen 1

und 2 Mol, vorzugsweise zwischen 1,1 und 1,5 Mol Sulfonsäurechlorid der Formel (V) ein.

Die Durchführung und Aufarbeitung kann bei Verfahren (b) analog zu Verfahren (a) erfolgen.

Das erfindungsgemäße Verfahren (c) wird vorzugsweise unter Verwendung von Verdünnungsmitteln durchgeführt. Als solche kommen praktisch alle inerten organischen Lösungsmittel in Betracht. Hierzu gehören insbesondere Alkohole, wie z.B. Ethanol, n- und iso-Propanol, Ether, wie z.B. Tetrahydrofuran, Dioxan und 1,2-Dimethoxyethan, Ester, wie z.B. Essigsäuremethylester und -ethylester sowie Nitrile, wie z.B. Acetonitril.

Die Reaktionstemperatur kann bei Verfahren (c) innerhalb eines größeren Bereiches variiert werden. Im allgemeinen arbeitet man zwischen -20°C und +50°C, vorzugsweise zwischen 0°C und 30°C. Verfahren (c) wird im allgemeinen bei Normaldruck durchgeführt.

Zur Durchführung des erfindungsgemäßen Verfahrens (c) setzt man je Mol Verbindung der Formel (I) im allgemeinen zwischen 0,9 und 1,2 Mol, vorzugsweise zwischen 0,95 und 1,1 Mol Metallverbindung ein.

Im allgemeinen werden die Verbindungen der Formel (I) und das Verdünnungsmittel vorgelegt und - gegebenenfalls unter leichter Außenkühlung - die Metallverbindung - gegebenenfalls im Verdünnungsmittel gelöst - zudosiert. Das Reaktionsgemisch wird bis zum Reaktionsende gerührt. Die salzartigen Produkte der Formel (I) fallen im allgemeinen kristallin an und können durch Absaugen isoliert werden.

Das erfindungsgemäße Verfahren (d) wird vorzugsweise unter Verwendung von Verdünnungsmitteln durchgeführt. Als solche kommen praktisch alle inerten organischen Lösungsmittel in Betracht. Hierzu gehören insbesondere Alkohole, wie Methanol, Ethanol, n- und iso-Propanol, Ether, wie Tetrahydrofuran, Dioxan und 1,2-Dimethoxyethan, Ester, wie Essigsäuremethylester und -ethylester sowie Ketone, wie Aceton, Methylethylketon und Methylisobutylketon.

Soweit die als Ausgangsstoffe verwendeten Säuren in wäßriger Lösung eingesetzt werden, kann vorteilhaft auch Essigsäureanhydrid als Verdünnungsmittel verwendet werden.

Die Reaktionstemperatur kann bei Verfahren (d) innerhalb eines größeren Bereiches variiert werden. Im allgemeinen arbeitet man zwischen -20°C und +50°C, vorzugsweise zwischen 0°C und 30°C. Verfahren (d) wird im allgemeinen bei Normaldruck durchgeführt.

Zur Durchführung des erfindungsgemäßen Verfahrens (d) setzt man je Mol Verbindung der Formel (I) im allgemeinen zwischen 1 und 10 Mol, vorzugsweise zwischen 1 und 5 Mol einer starken Säure ein.

Im allgemeinen werden die Verbindungen der Formel (I) und das Verdünnungsmittel vorgelegt und - gegebenenfalls unter

leichter Außenkühlung - die starke Säure zudosiert. Das Reaktionsgemisch wird bis zum Reaktionsende gerührt. Die 1:1-Addukte fallen im allgemeinen kristallin an und können durch Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können zur Bekämpfung monokotyler und dikotyler Unkräuter in monokotylen und dikotylen Kulturen im Vorauflauf- und Nachauflauf-Verfahren eingesetzt werden.

Eine Kontrolle keimender, auflaufender und bereits etablierter Unkräuter in Dauerkulturen ist mit den erfindungsgemäßen Wirkstoffen ebenso möglich, wie die totale Vegetationsbekämpfung auf Nichtkulturland.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. N-(2-Benzthiazolyl)-N,N'-dimethyl-harnstoff, 3-(3-Chlor-4-methylphenyl)-1,1-dimethylharnstoff, 3-(4-Isopropylphenyl)-1,1-dimethylharnstoff, 4-Amino-6-(1,1-dimethyl-ethyl)-3-methylthio-1,2,4-triazin-5(4H)-on, 4-Amino-6-(1,1-dimethyl-ethyl)-3-ethylthio-1,2,4-triazin-5(4H)-on, 1-Amino-6-ethylthio-3-(2,2-dimethylpropyl)-1,3,5-triazin-2,4-(1H,3H)-dion, 4-Amino-3-methyl-6-phenyl-1,2,4-triazin-5(4H)-on, 2-Chlor-4-ethylamino-6-isopropyl-amino-1,3,5-triazin, das R-Enantiomere des 2-[4-(3,5-Dichlor-pridin-2-oxy)-phenoxy]-propionsäure-(trimethylsilyl)-methylesters, das R-Enantiomere des 2-[4-(3,5-Dichlor-pyridyl-2-oxy)-phenoxy]-propionsäure-(2-benzyloxy)-ethylesters, 2,4-Dichlorphenoxyessigsäure, 2-(2,4-Dichlor-phenoxy)-propionsäure, 4-Chlor-2-methyl-phenoxy-essigsäure, 2-(2-Methyl-4-chlor-phenoxy)-propionsäure, 3,5-Dijod-4-hydroxy-benzonitril, 3,5-Dibrom-4-hydroxy-benzonitril sowie Diphenylether und Phenylpyridazine, wie z.B. Pyridate. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 5 kg pro ha.

Verbindungen der Formel (I) zeigen auch fungizide Wirkung.

Die Herstellung und die Verwendung der erfindungsgemäßen Wirkstoffe geht aus den nachfolgenden Beispielen hervor.

Herstellungsbeispiele:

Beispiel 1

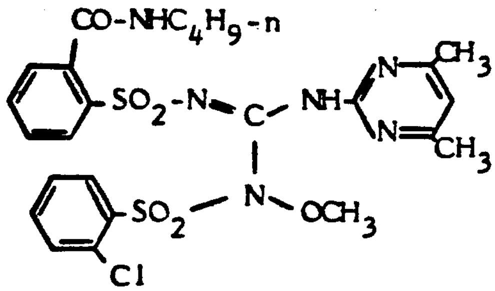

(Verfahren (a))

2,5 g (0,012 Mol) 2-Chlor-benzolsulfonsäurechlorid werden zu einer Mischung aus 5,0 g (0,011 Mol) N'-(4,6-Dimethyl-pyrimidin-2-yl)-N"-methoxy-N"'-(2-butylaminocarbonyl-benzol-sulfonyl)-guanidin, 2,0 g (0,016 Mol) 4-Dimethylamino-pyridin und 30 ml Acetonitril gegeben. Das Reaktionsgemisch wird 3 Stunden unter Rückfluß zum Sieden erhitzt und anschließend eingeengt. Der Rückstand wird in 100 ml Methylenchlorid aufgenommen. Diese Lösung wird mit 100 ml 5 %iger Salzsäure und mit 100 ml Wasser gewaschen, getrocknet, filtriert und eingeengt.

Man erhält 1,8 g (30 % der Theorie) N'-(4,6-Dimethyl-pyrimidin-2-yl)-N"-methoxy-N"-(2-chlor-benzolsulfonyl)-N"'-(2-butylaminocarbonyl-benzolsulfonyl)-guanidin als kristallinen Rückstand vom Schmelzpunkt 157°C.

Beispiel 2

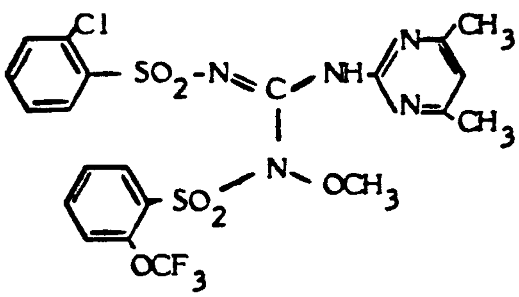

(Verfahren (b))

Eine Lösung von 5,3 g (0,025 Mol) 2-Chlor-benzolsulfonsäure-chlorid in 10 ml Methylenchlorid wird zu einer auf 0°C gekühlten Mischung aus 10,5 g (0,025 Mol) N'-(4,6-Dimethyl-pyrimidin-2-yl)-N"-methoxy-N"-(2-trifluormethoxy-benzolsulfonyl)-guanidin, 3,1 g (0,0275 Mol) Diazabicyclooctan (DABCO) und 100 ml Methylenchlorid unter Rühren tropfenweise gegeben. Das Reaktionsgemisch wird 15 Stunden bei 20°C gerührt, anschließend mit 2n-Salzsäure und mit Wasser gewaschen, getrocknet, filtriert und eingeengt. Der Rückstand wird durch Chromatographie über eine Kieselgelsäule gereinigt.

Man erhält 1,0 g (7 % der Theorie) N'-(4,6-Dimethyl-pyrimidin-2-yl)-N"-methoxy-N"-(2-trifluormethoxy-benzolsulfonyl)-N"'-(2-chlor-benzolsulfonyl)-guanidin vom Schmelzpunkt 160°C.

Beispiel 3

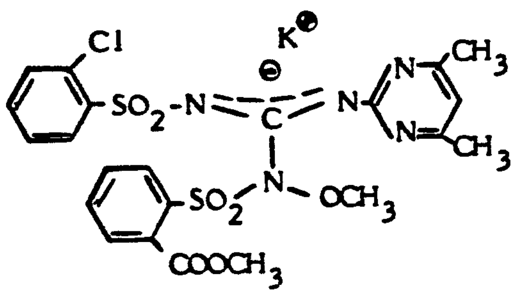

(Verfahren (c))

Eine Lösung von 0,9 g (0,01 Mol) Kaliumethanolat in 15 ml Ethanol wird zu einer Mischung aus 5,7 g (0,01 Mol) N'-(4,6-Dimethyl-pyrimidin-2-yl)-N"-methoxy-N"-(2-methoxycarbonyl-benzolsulfonyl)-N"'-(2-chlor-benzolsulfonyl)-guanidin und 15 ml Ethanol gegeben und das Reaktionsgemisch wird 5 Stunden bei 20°C gerührt. Das kristallin angefallene Produkt wird durch Absaugen isoliert.

Man erhält 1,9 g (31 % der Theorie) N'-(4,6-Dimethyl-pyrimidin-2-yl)-N"-methoxy-N"-(2-methoxycarbonyl-benzolsulfonyl)-N"'-(2-chlor-benzolsulfonyl)-guanidin-Kaliumsalz vom Schmelzpunkt 161°C.

Beispiel 4

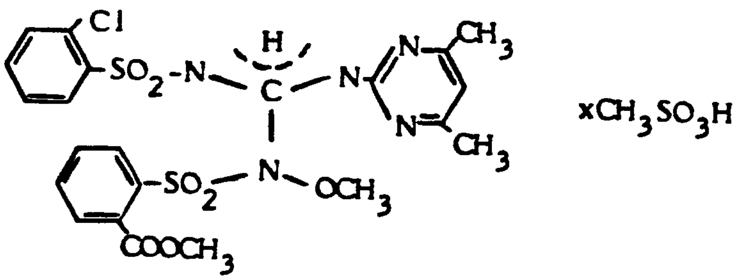

(Verfahren (d))

Eine Mischung aus 1,5 g (0,003 Mol) N'-(4,6-Dimethyl-pyrimidin-2-yl)-N"-methoxy-N"-(2-methoxycarbonyl-benzolsulfonyl)-N"'-(2-chlor-benzolsulfonyl)-guanidin, 0,3 g (0,003 Mol) Methansulfonsäure und 15 ml Aceton wird 2 Tage bei 20°C gerührt. Das kristallin angefallene Produkt wird durch Absaugen isoliert.

Man erhält 1,4 g (24 % der Theorie) des 1:1-Adduktes aus N'-(4,6-Dimethyl-pyrimidin-2-yl)-N"-methoxy-N"-(2-methoxycarbonyl-benzolsulfonyl)-N"'-(2-chlor-benzolsulfonyl)-guanidin und Methansulfonsäure vom Schmelzpunkt 131°C.

Nach den in den vorausgehenden Beispielen exemplarisch beschriebenen Verfahren können die in der nachstehenden Tabelle aufgeführten Verbindungen der Formel (I) hergestellt werden:

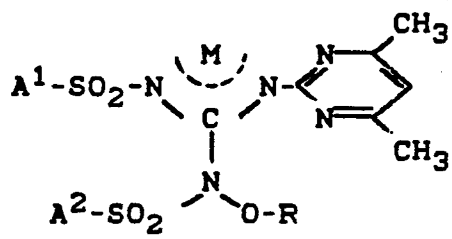

(I)

Tabelle 1

| Beisp. Nr. | $A^1$ | $A^2$ | R | M | Schmelzpunkt (°C) |
|---|---|---|---|---|---|
| 5 | 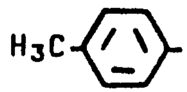 | 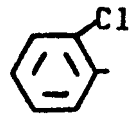 | $CH_3$ | H | 85 |
| 6 | 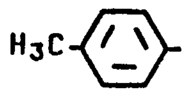 | 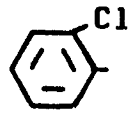 | 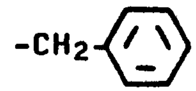 | H | 168 |
| 7 | 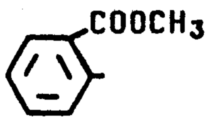 | 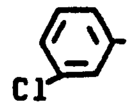 | $CH_3$ | H | 158 |
| 8 | 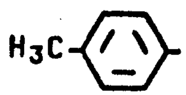 | 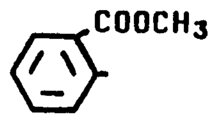 | $CH_3$ | H | 150 |
| 9 | 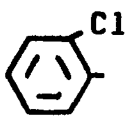 | 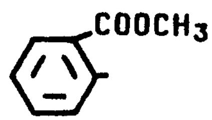 | $CH_3$ | H | 149 |

Tabelle 1 - Fortsetzung

| Beisp. Nr. | $A^1$ | $A^2$ | R | M | Schmelzpunkt (°C) |
|---|---|---|---|---|---|
| 10 | 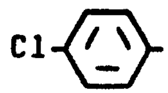 | 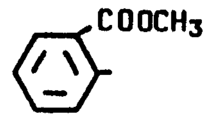 | $CH_3$ | H | 145 |
| 11 | 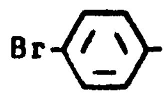 | 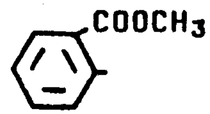 | $CH_3$ | H | 153 |
| 12 | 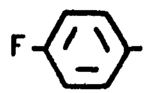 | 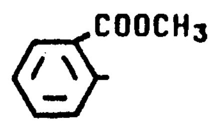 | $CH_3$ | H | 154 |
| 13 | 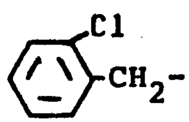 | 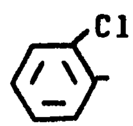 | 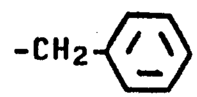 | H | teilkristallin |
| 14 | 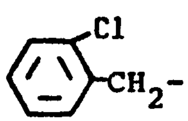 | 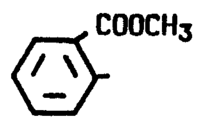 | $CH_3$ | H | 160 |
| 15 | 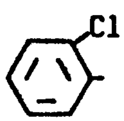 | 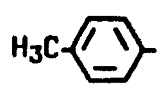 | $C_4H_9$-n | H | amorph |

<u>Tabelle 1 - Fortsetzung</u>

| Beisp. Nr. | $A^1$ | $A^2$ | R | M | Schmelz- punkt (°C) |
|---|---|---|---|---|---|
| 16 | 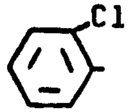 | 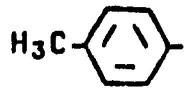 | $C_8H_{17}$-n | H | |
| 17 | 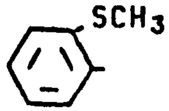 | 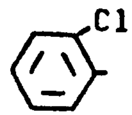 | 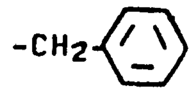 | H | 156 |
| 18 | 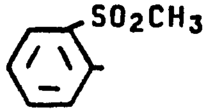 | 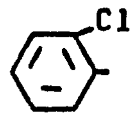 | 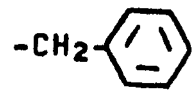 | H | 174 |
| 19 | 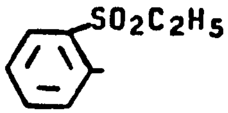 | 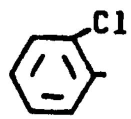 | 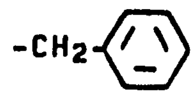 | H | |
| 20 | 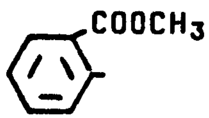 | 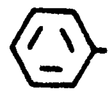 | 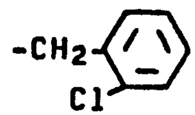 | H | |
| 21 | 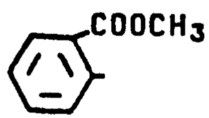 | 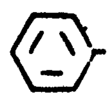 | 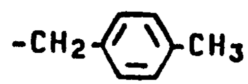 | H | |

Tabelle 1 - Fortsetzung

| Beisp. Nr. | $A^1$ | $A^2$ | R | M | Schmelz-punkt (°C) |
|---|---|---|---|---|---|
| 22 | 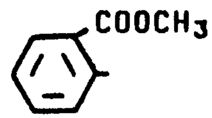 | 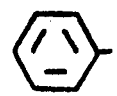 | 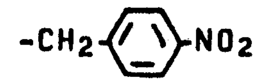 | H | |
| 23 | 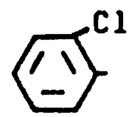 | 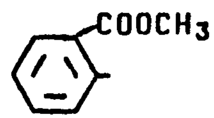 | 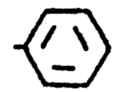 | H | |
| 24 | 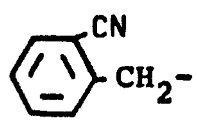 | 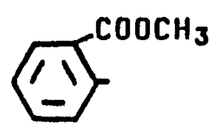 | 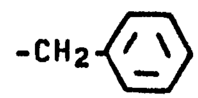 | H | |
| 25 | 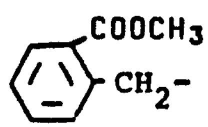 | 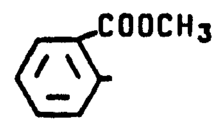 | 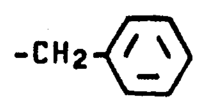 | H | |
| 26 | 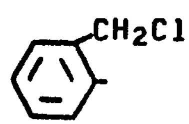 | 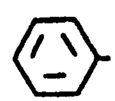 | $-CH_2COOC_2H_5$ | H | |
| 27 | 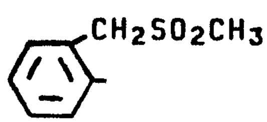 | 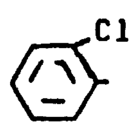 | 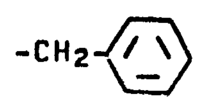 | H | |

Tabelle 1 - Fortsetzung

| Beisp. Nr. | $A^1$ | $A^2$ | R | M | Schmelzpunkt (°C) |
|---|---|---|---|---|---|
| 28 | 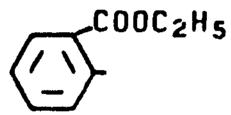 | 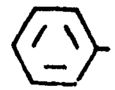 | $C_2H_5$ | H | |
| 29 | 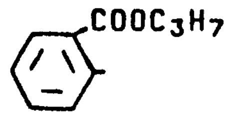 | 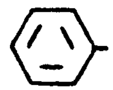 | $C_2H_5$ | H | |
| 30 | 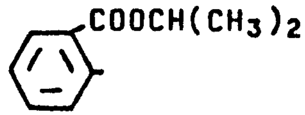 | 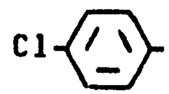 | $CH_3$ | H | |
| 31 | 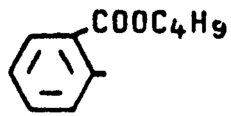 | 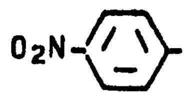 | $C_2H_5$ | H | |
| 32 | 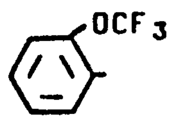 | 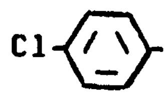 | $C_3H_7$ | H | |
| 33 | 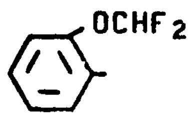 | 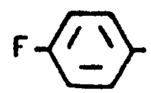 | $C_3H_7$ | H | |

<u>Tabelle 1 - Fortsetzung</u>

| Beisp. Nr. | $A^1$ | $A^2$ | R | M | Schmelzpunkt (°C) |
|---|---|---|---|---|---|
| 34 | 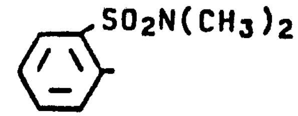 | 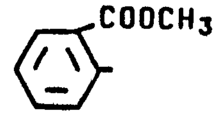 | $-CH_2CH(CH_3)_2$ | H | |
| 35 | 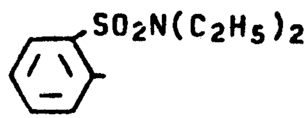 | 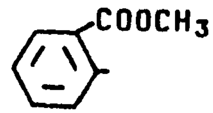 | $-CH_2CH(CH_3)_2$ | H | |
| 36 | 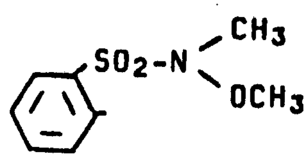 | 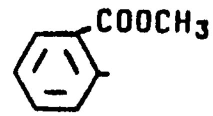 | $-CH_2CH(CH_3)_2$ | H | |
| 37 | 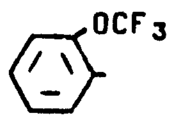 | 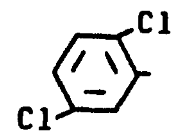 | $-CH_2CH_2-$ 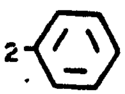 | H | |
| 38 | 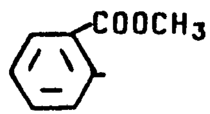 | 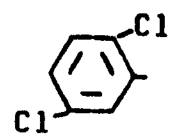 | $-CH_2CH_2-$ 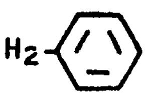 | H | |

Tabelle 1 - Fortsetzung

| Beisp. Nr. | $A^1$ | $A^2$ | R | M | Schmelz-punkt (°C) |
|---|---|---|---|---|---|
| 39 | 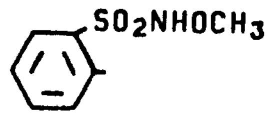 | 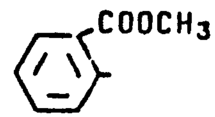 | $CH_3$ | H | |
| 40 | 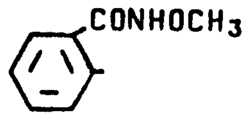 | 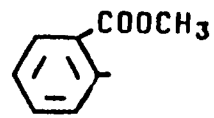 | $CH_3$ | H | |
| 41 | 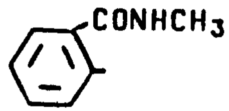 | 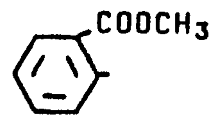 | $CH_3$ | H | |
| 42 | 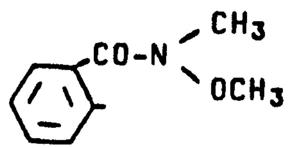 | 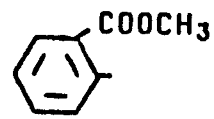 | $CH_3$ | H | |
| 43 | 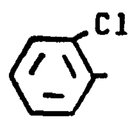 | 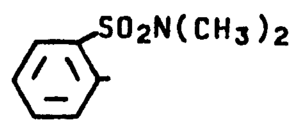 | $-CH_2CH(CH_3)_2$ | H | |
| 44 | 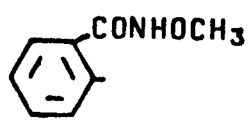 | 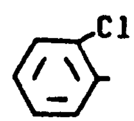 | $CH_3$ | H | 155 |

Tabelle 1 - Fortsetzung

| Beisp. Nr. | $A^1$ | $A^2$ | R | M | Schmelz-punkt (°C) |
|---|---|---|---|---|---|
| 45 | 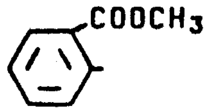 | 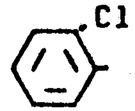 | $CH_3$ | H | 152 |
| 46 | 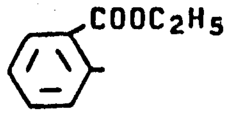 | 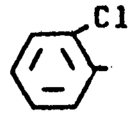 | $CH_3$ | H | |
| 47 | 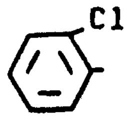 | 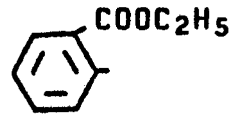 | $CH_3$ | H | 161 |
| 48 | 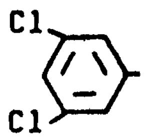 | 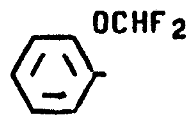 | $CH_3$ | H | 171 |
| 49 | 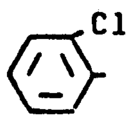 | 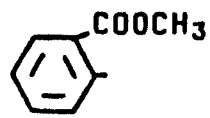 | $-CH_2CH(CH_3)_2$ | H | 156 |
| 50 | 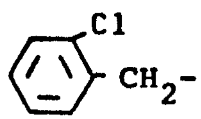 | 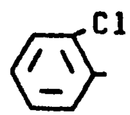 | $CH_3$ | H | 176 |

Tabelle 1 - Fortsetzung

| Beisp. Nr. | $A^1$ | $A^2$ | R | M | Schmelzpunkt (°C) |
|---|---|---|---|---|---|
| 51 | 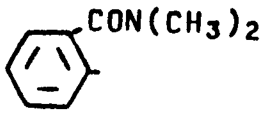 | 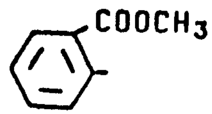 | $CH_3$ | H | |
| 52 | 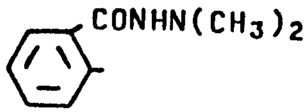 | 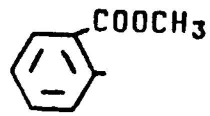 | $CH_3$ | H | |
| 53 | 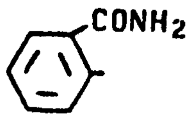 | 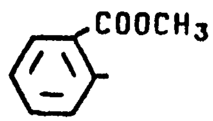 | $CH_3$ | H | |
| 54 | 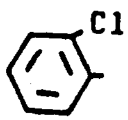 | 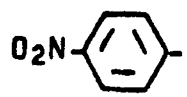 | $CH_3$ | H | |
| 55 | 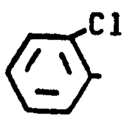 | 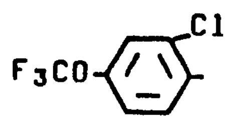 | $CH_3$ | H | |
| 56 | 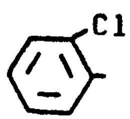 | 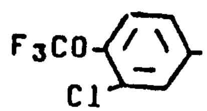 | 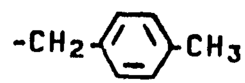 | H | |

Tabelle 1 - Fortsetzung

| Beisp. Nr. | $A^1$ | $A^2$ | R | M | Schmelzpunkt (°C) |
|---|---|---|---|---|---|
| 57 | 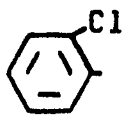 | 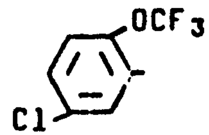 | 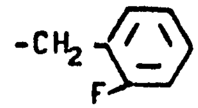 | H | |
| 58 | 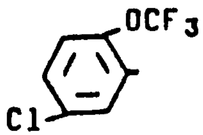 | 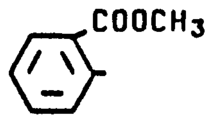 | 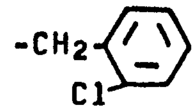 | H | |
| 59 | 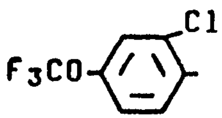 | 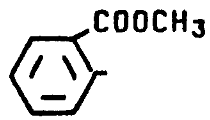 | $C_2H_5$ | H | |
| 60 | 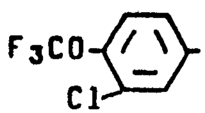 | 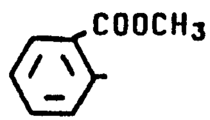 | $C_2H_5$ | H | |
| 61 | 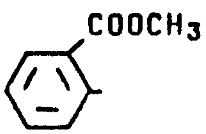 | 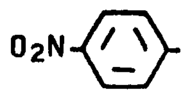 | $C_3H_7$ | H | |

Tabelle 1 - Fortsetzung

| Beisp. Nr. | $A^1$ | $A^2$ | R | M | Schmelzpunkt (°C) |
|---|---|---|---|---|---|
| 62 | 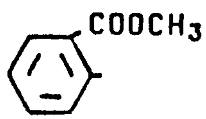 | 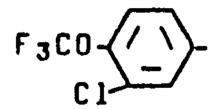 | 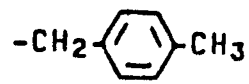 | H | |
| 63 | 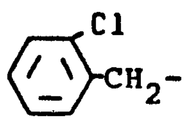 | 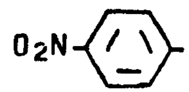 | $CH_3$ | H | |
| 64 | 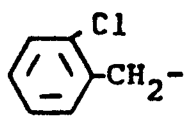 | 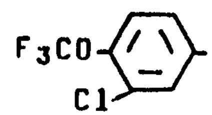 | $CH_3$ | H | |
| 65 | 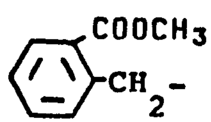 | 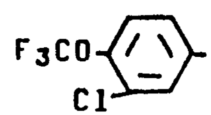 | 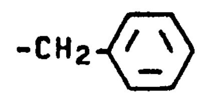 | H | |

Tabelle 1 - Fortsetzung

| Beisp. Nr. | $A^1$ | $A^2$ | R | M | Schmelz-punkt (°C) |
|---|---|---|---|---|---|
| 66 | 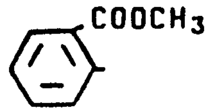 | 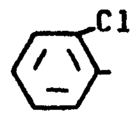 | $-CH_2-CH(CH_3)_2$ | H | 119 |
| 67 | 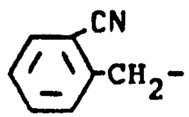 | 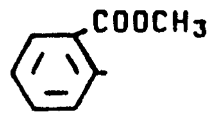 | $-CH_3$ | H | 183-184 |
| 68 | 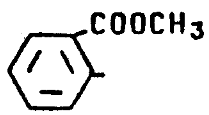 | 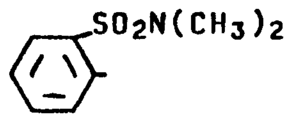 | $-CH_2-CH(CH_3)_2$ | H | 157 |
| 69 | 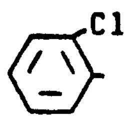 | 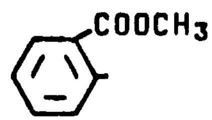 | $-CH_2-COOC_2H_5$ | H | 103 - 104 |
| 70 | 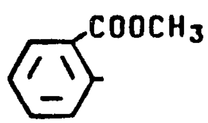 | 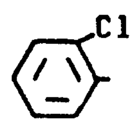 | $-CH_2-COOC_2H_5$ | H | 142 - 143 |
| 71 | 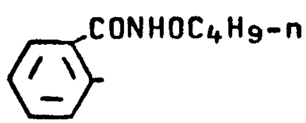 | 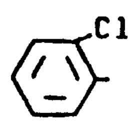 | $-C_8H_{17}-n$ | H | (amorph) |

Tabelle 1 - Fortsetzung

| Beisp. Nr. | $A^1$ | $A^2$ | R | M | Schmelz-punkt (°C) |
|---|---|---|---|---|---|
| 72 | 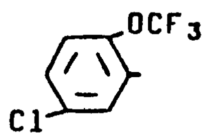 | 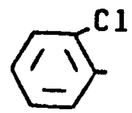 | 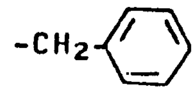 | H | (amorph) |
| 73 | 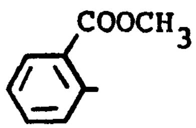 | $CH_3CH_2CH_2CH_2-$ | $-CH_3$ | H | (amorph) |
| 74 | 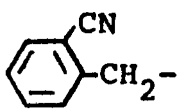 | 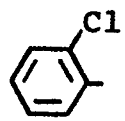 | $-CH_3$ | H | 156-159 |
| 75 | 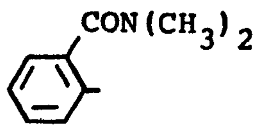 | 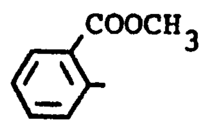 | $-C_2H_5$ | H | 120 (Zers.) |
| 76 | 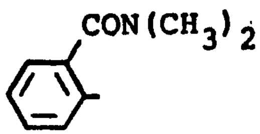 | 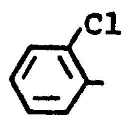 | $-C_2H_5$ | H | 153-155 |
| 77 | 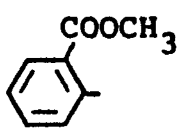 | 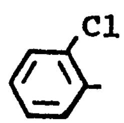 | 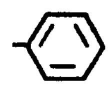 | H | 125-126 |

Tabelle 1 - Fortsetzung

| Bsp. Nr. | $A^1$ | $A^2$ | R | M | Schmelz-punkt (°C) |
|---|---|---|---|---|---|
| 78 | 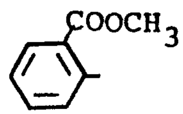 | 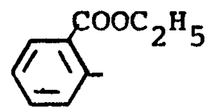 | $-CH_3$ | H | 165 |
| 79 | 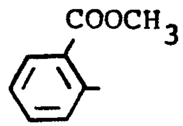 | 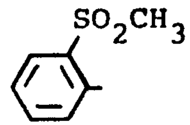 | $-CH_3$ | H | 173 |
| 80 | 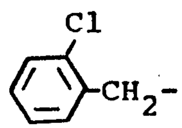 | 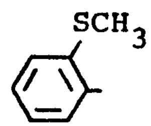 | $-CH_3$ | H | 171 |
| 81 | 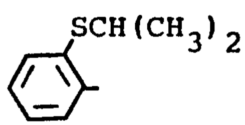 | 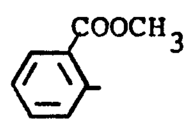 | $-CH_3$ | H | 145 |
| 82 | 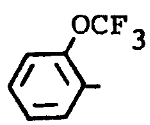 | $CH_3CH_2CH_2CH_2-$ | $-CH_3$ | H | 73 |
| 83 | 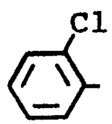 | $CH_3CH_2CH_2CH_2-$ | $-CH_3$ | H | 127 |
| 84 | 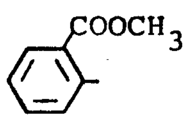 | $C_8F_{17}-$ | $-CH_3$ | H | 135 |

Tabelle 1 - Fortsetzung

| Bsp. Nr. | $A^1$ | $A^2$ | R | M | Schmelzpunkt (°C) |
|---|---|---|---|---|---|
| 85 | 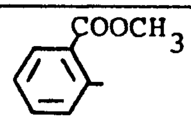 | $(CH_3)_2N-$ | $-CH_3$ | H | 125 |
| 86 | 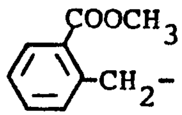 | 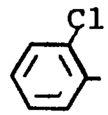 | $-CH_2-$ 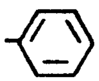 | H | 154 |
| 87 | 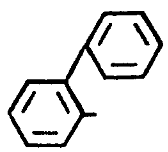 | $n-C_4H_9-$ | $-CH_3$ | H | 145-147 |
| 88 | $F_3C-$ | 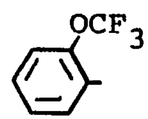 | $-CH_3$ | H | amorph |
| 89 | 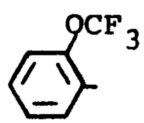 | $(CH_3)_2N-$ | $-CH_3$ | H | 143 - 145 |
| 90 | 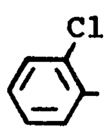 | $(CH_3)_2N-$ | $-CH_3$ | H | 152 - 154 |
| 91 | $F_3CO-$ 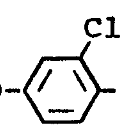 | $n-C_4H_9-$ | $-CH_3$ | H | 80 - 84 |

Tabelle 1 - Fortsetzung

| Bsp.-Nr. | $A^1$ | $A^2$ | R | M | Schmelzpunkt (°C) |
|---|---|---|---|---|---|
| 92 | 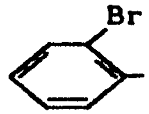 | $(CH_3)_2N-$ | $-CH_3$ | H | 147 - 148 |
| 93 | 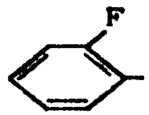 | $(CH_3)_2N-$ | $-CH_3$ | H | amorph |
| 94 | 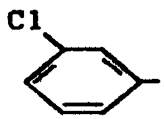 | 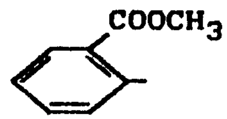 | $-CH_3$ | H | 80 |
| 95 | 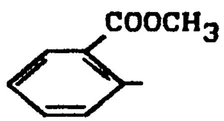 | 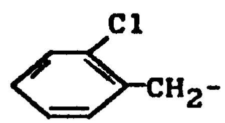 | $-CH_3$ | H | 77 - 79 |
| 96 | 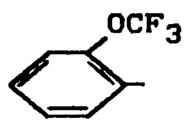 | 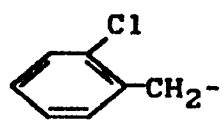 | $-CH_3$ | H | 137 - 140 |

14a) p-Toluolsulfonsäure-Salz von Beispiel 14, vom Schmelzpunkt 146 °C

14b) Methansulfonsäure-Salz von Beispiel 14, vom Schmelzpunkt 135 - 138 °C

2a) Methansulfonsäure-Salz von Beispiel 2, vom Schmelzpunkt 178 °C (Zers.)

50a) Methansulfonsäure-Salz von Beispiel 50, vom Schmelzpunkt 177 °C

80a) Methansulfonsäure-Salz von Beispiel 80, vom Schmelzpunkt 170 °C

80b) Schwefelsäure-Salz von Beispiel 80, vom Schmelzpunkt 149 - 152 °C.

Herstellung der Ausgangsstoffe der Formel (II)

Beispiel (II-1)

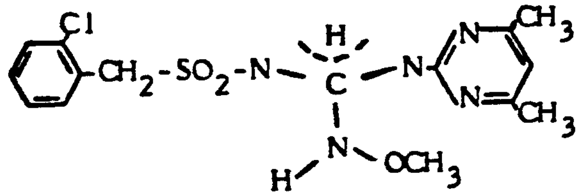

Eine Lösung von 16,8 g (0,15 Mol) Diazabicyclooctan in 50 ml Methylenchlorid wird zu einer auf 0°C gekühlten Mischung aus 9,8 g (0,05 Mol) N'-(4,6-Dimethyl-pyrimidin-2-yl)-N"-methoxy-guanidin, 28,2 g (0,12 Mol) 2-Chlorphenylmethansulfonsäurechlorid und 50 ml Methylenchlorid unter Rühren tropfenweise gegeben. Das Reaktionsgemisch wird 2 Stunden bei 10°C und weitere 15 Stunden bei 20°C gerührt. Anschließend werden 50 ml 2N Salzsäure dazugegeben, nach Durchschütteln die organische Phase abgetrennt, mit Wasser gewaschen, getrocknet, filtriert und eingeengt. Der ölige Rückstand wird mit Isopropanol zur Kristallisation gebracht.

Man erhält 3,0 g (12 % der Theorie) N'-(4,6-Dimethyl-pyrimidin-2-yl)-N"-methoxy-N"'-(2-chlor-benzylsulfonyl)-guanidin vom Schmelzpunkt 130°C.

Beispiel (II-2)

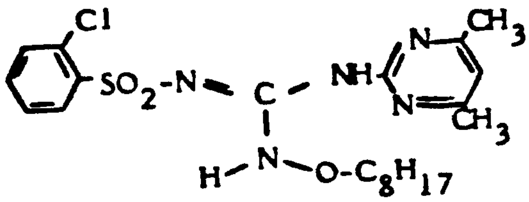

Eine Mischung aus 13,8 g (0,025 Mol) N'-(4,6-Dimethyl-pyrimidin-2-yl)-N"-methoxy-N",N"'-bis-(2-chlor-benzol-sulfonyl)-guanidin, 3,7 g (0,026 Mol) O-Octyl-hydroxylamin und 80 ml Ethanol wird 15 Stunden unter Rückfluß zum Sieden erhitzt. Dann wird im Wasserstrahlvakuum (ca. 15 mbar) eingeengt, das Produkt durch Anreiben zur Kristallisation gebracht und durch Absaugen isoliert.

Man erhält 3,2 g (27 % der Theorie) N'-(4,6-Dimethyl-pyrimidin-2-yl)-N"-octyloxy-N"'-(2-chlor-benzol-sulfonyl)-guanidin vom Schmelzpunkt 55°C.

Beispiel (II-3)

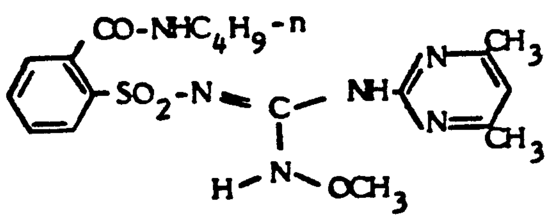

4,0 g (0,025 Mol) Butylamin werden bei 20°C - 30°C zu einer Mischung aus 9,1 g (0,025 Mol) der Verbindung nachstehender Formel

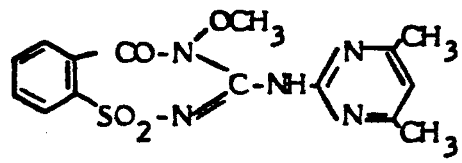

sowie 60 ml Diethylether und 5 ml Dioxan unter Rühren gegeben. Das Reaktionsgemisch wird 15 Stunden bei 20°C gerührt und das kristallin angefallene Produkt durch Absaugen isoliert.

Man erhält 8,1 g (75 % der Theorie) N'-(4,6-Dimethyl-pyrimidin-2-yl)-N''-methoxy-N'''-(2-butylaminocarbonyl-benzolsulfonyl)-guanidin vom Schmelzpunkt 169°C.

Beispiel (II-4)

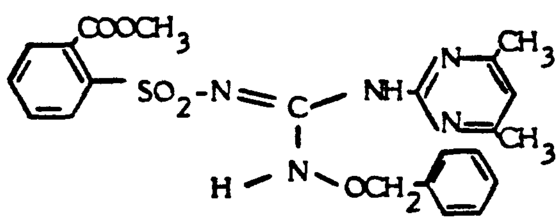

Eine Mischung aus 8,8 g (0,02 Mol) der Verbindung nachstehender Formel

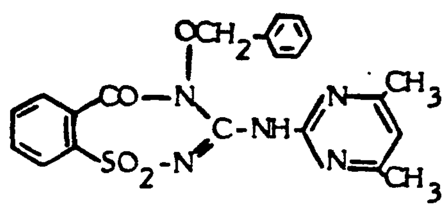

sowie 2,2 g (0,04 Mol) Natrium-methylat und 70 ml Methanol wird 8 Stunden unter Rückfluß zum Sieden erhitzt. Nach Abkühlen auf -10°C wird abgesaugt, der Feststoff in 200 ml Wasser gelöst, die Lösung filtriert und das Filtrat mit konz. Salzsäure angesäuert. Das kristallin angefallene Produkt wird durch Absaugen isoliert.

Man erhält 3,2 g (34 % der Theorie) N'-(4,6-Dimethyl-pyrimidin-2-yl)-N"-benzyloxy-N"'-(2-methoxycarbonyl-benzolsulfonyl)-guanidin vom Schmelzpunkt 128°C.

Beispiel (II-5)

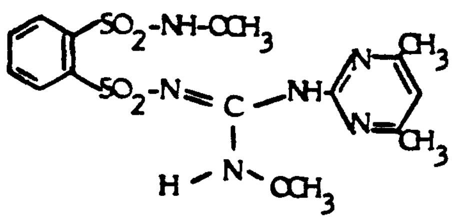

6,9 g (0,025 Mol) Benzol-1,2-disulfonsäure-dichlorid werden zu einer auf -20°C gekühlten Mischung aus 4,9 g (0,025 Mol) N'-(4,6-Dimethyl-pyrimidin-2-yl)-N"-methoxy-guanidin, 6,0 g (0,0375 Mol) Pyridin und 50 ml Methylenchlorid tropfenweise gegeben. Das Reaktionsgemisch wird 3 Stunden bei -20°C und weitere 15 Stunden bei +20°C gerührt. Anschließend werden 1,2 g (0,025 Mol) O-Methyl-hydroxylamin zur Reaktionsmischung gegeben und weitere 6 Stunden bei 20°C gerührt. Dann wird filtriert, eingeengt und der Rückstand durch Anreiben mit Ethanol zur Kristallisation gebracht.

Man erhält 7,2 g (72 % der Theorie) N'-(4,6-Dimethyl-pyrimidin-2-yl)-N"-methoxy-N"'-(2-methoxyamino-sulfonyl-benzolsulfonyl)-guanidin vom Schmelzpunkt 208°C.

Analog Beispiel (II-1) bis Beispiel (II-5) können die in der nachstehenden Tabelle aufgeführten Verbindungen der Formel (II) hergestellt werden:

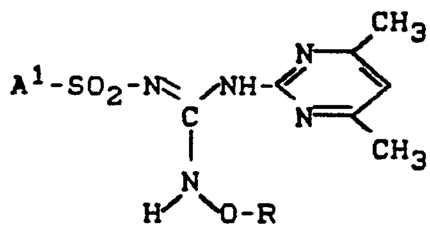

(II)

Tabelle 2

| Beisp. Nr. | A [1] | R | Schmelz-punkt (°C) |
|---|---|---|---|
| II-6 | 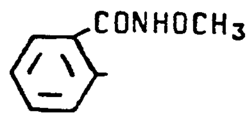 | $-CH_3$ | 151 |
| II-7 | 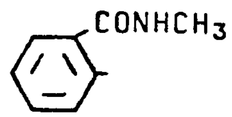 | $-CH_3$ | 177 |
| II-8 | 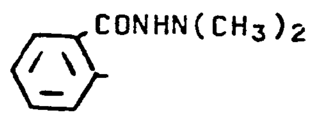 | $-CH_3$ | 178 |
| II-9 | 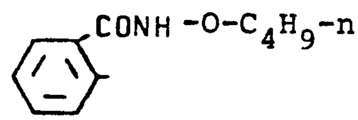 | $-C_8H_{17}$-n | (amorph) |
| II-10 | 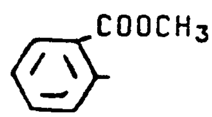 | $-CH_3$ | 132 |
| II-11 | 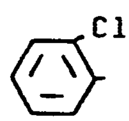 | $-CH_3$ | 142 |
| II-12 | 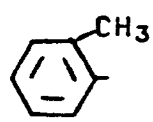 | $-CH_3$ | 114 |
| II-13 | 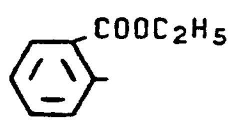 | $-CH_3$ | 125 |
| II-14 | 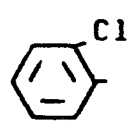 | $-CH_2COOCH_3$ | 124 |

Tabelle 2 - Fortsetzung

| Beisp. Nr. | $A^1$ | R | Schmelz-punkt (°C) |
|---|---|---|---|
| II-15 | 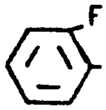 | $-CH_3$ | 160 |
| II-16 | 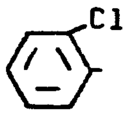 | $-CH_2-COOC_3H_7$-i | 112 |
| II-17 | 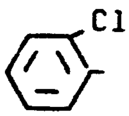 | $-CH(CH_3)-COOC_2H_5$ | 128 |
| II-18 | 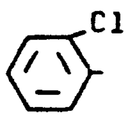 | $-CH(CH_3)-COOCH_3$ | 124 |
| II-19 | 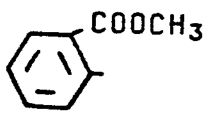 | $-CH_2-COOC_2H_5$ | 138 - 139 |
| II-20 | 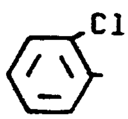 | $-CH_2-$ 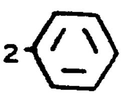 | 169 |
| II-21 | 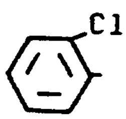 | $-C_4H_9$-n | 116 |
| II-22 | 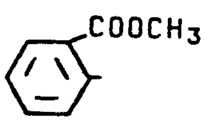 | $-C_8H_{17}$-n | 105 |

Tabelle 2 - Fortsetzung

| Beisp. Nr. | $A^1$ | R | Schmelzpunkt (°C) |
|---|---|---|---|
| II-23 | 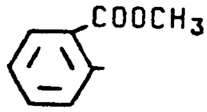 | $-C_4H_9$-n | 95 |
| II-24 | 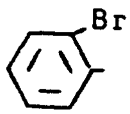 | $-CH_3$ | 166 |
| II-25 | 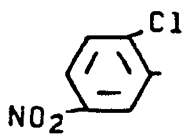 | $-CH_3$ | 160 |
| II-26 | 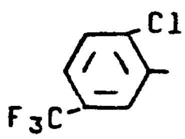 | $-CH_3$ | 134 |
| II-27 | 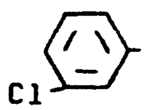 | $-CH_3$ | |
| II-28 | 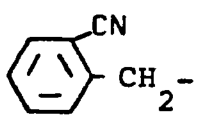 | $-CH_3$ | 166 - 168 |
| II-29 | 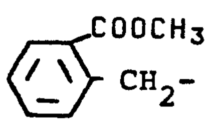 | $-CH_3$ | 117 - 125 |
| II-30 | 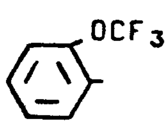 | $-CH_3$ | 128 |

Tabelle 2 - Fortsetzung

| Beisp. Nr. | $A^1$ | R | Schmelzpunkt (°C) |
|---|---|---|---|
| II-31 | 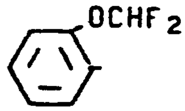 | $-CH_3$ | 138 |
| II-32 | 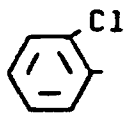 | $-CH_2-COOC_2H_5$ | 109 - 110 |
| II-33 | 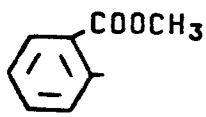 | $-CH_2-COOC_3H_7-i$ | 82 - 83 |
| II-34 | 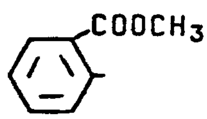 | $-\underset{\mid \atop CH_3}{CH}-COOCH_3$ | 99 - 100 |
| II-35 | 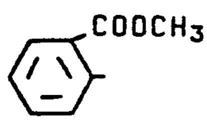 | $-CH_2-COOCH_3$ | 101 - 102 |
| II-36 | 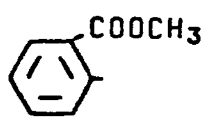 | $-\underset{\mid \atop CH_3}{CH}-COOC_2H_5$ | 54 - 56 |
| II-37 | 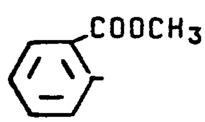 | $-CH_2-COOH$ | 138 - 140 (Zers.) |
| II-38 | 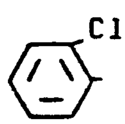 | 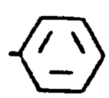 | 146 - 149 |

Tabelle 2 - Fortsetzung

| Beisp. Nr. | $A^1$ | R | Schmelzpunkt (°C) |
|---|---|---|---|
| II-39 | 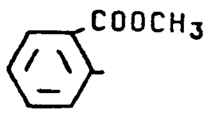 | 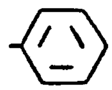 | 143 - 145 |
| II-40 | 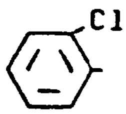 | $-CH_2-CH(CH_3)_2$ | 105 |
| II-41 | 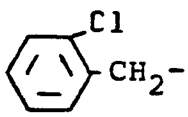 | 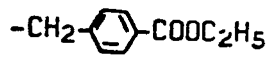 | 118 |
| II-42 | 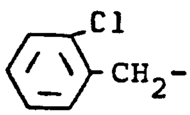 | $-CH_2-CH_2-CH_2-Cl$ | 139 - 140 |
| II-43 | 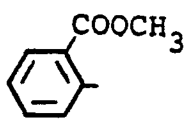 | 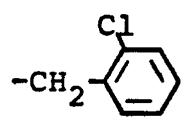 | Öl |
| II-44 | 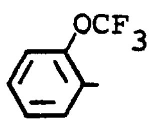 | $-C_2H_5$ | 115 |
| II-45 | 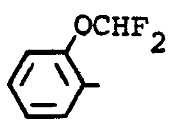 | $-C_2H_5$ | 136 |
| II-46 | 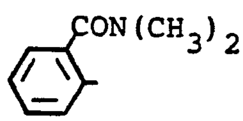 | $-CH_3$ | 153 |
| II-47 | 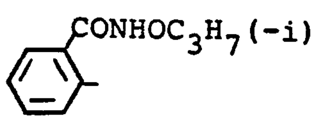 | $-CH_3$ | 178 |

Tabelle 2 - Fortsetzung

| Beisp. Nr. | $A^1$ | R | Schmelz-punkt (°C) |
|---|---|---|---|
| II-48 | 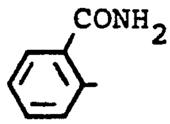 | $-CH_3$ | 222 |
| II-49 | 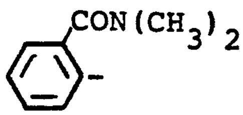 | $-C_2H_5$ | 143-148 |
| II-50 | 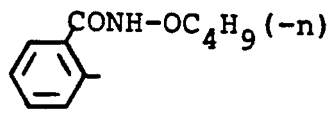 | 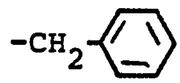 | δ1 |
| II-51 | 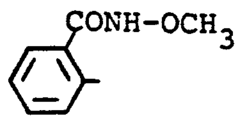 | $-C_4H_9$(-n) | δ1 |
| II-52 | 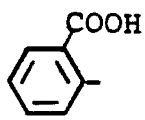 | 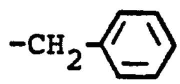 | 85 |
| II-53 | 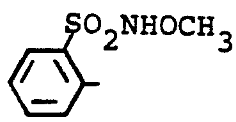 | 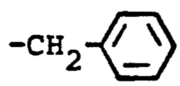 | 154 |
| II-54 | 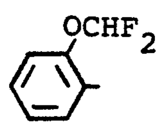 | 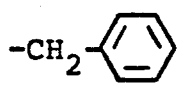 | 242 (Zers.) |
| II-55 | 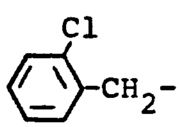 | 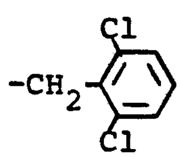 | 156 |
| II-56 | 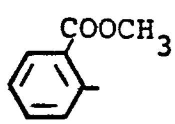 | $-CH_2CH_2CH_2Cl$ | 111 |

Tabelle 2 - Fortsetzung

| Beisp.-Nr. | $A^1$ | R | Schmelzpunkt (°C) |
|---|---|---|---|
| II-57 | 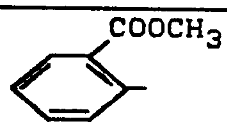 | $-CH_2-$ 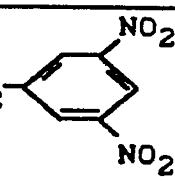 | 137 |
| II-58 | 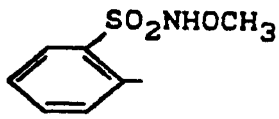 | $-CH_2-$ 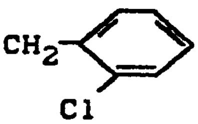 | 179 - 183 |
| II-59 | 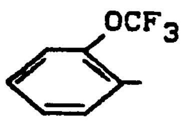 | $-CH_2-$ 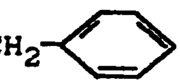 | 141 |
| II-60 | $CF_3-$ | $-CH_3$ | 105 - 108 |
| II-61 | $CH_3-$ | $-CH_3$ | amorph |
| II-62 | 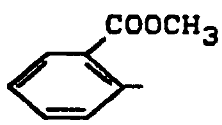 | $-CH_2-CH(CH_3)_2$ | 111 |
| II-63 | 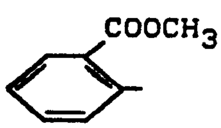 | $-\underset{CH_3}{\underset{\mid}{CH}}-CH_2-CH_3$ | 105 |

Tabelle 2 - Fortsetzung

| Beisp.-Nr. | $A^1$ | R | Schmelzpunkt (°C) |
|---|---|---|---|
| II-64 | 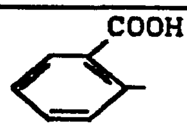 | $-CH_3$ | 148 |
| II-65 | 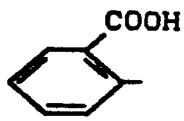 | $-C_4H_9$(-n) | 126 |
| II-66 | 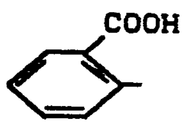 | $-C_8H_{17}$(-n) | 120 |
| II-67 | 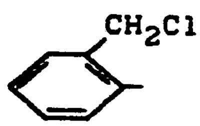 | $-CH_3$ | 101 - 103 |
| II-68 | 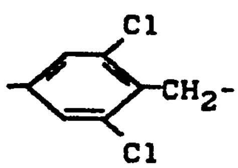 | $-CH_3$ | 168 |

Herstellung von Ausgangsstoffen der Formel (IV)

Beispiel (IV-1)

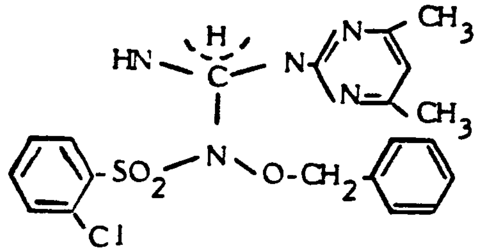

5,3 g (0,025 Mol) 2-Chlor-benzolsulfonsäurechlorid werden bei 20°C zu einer Mischung aus 6,8 g (0,025 Mol) N'-(4,6-Dimethyl-pyrimidin-2-yl)-N"-benzyloxy-guanidin, 2,6 g (0,025 Mol) Triethylamin und 50 ml Tetrahydrofuran unter Rühren gegeben. Das Reaktionsgemisch wird 15 Stunden bei 20°C gerührt, mit 250 ml Methylenchlorid verdünnt, mit Wasser gewaschen, getrocknet, filtriert und eingeengt. Der Rückstand wird mit Ethanol verrieben und das kristallin angefallene Produkt durch Absaugen isoliert.

Man erhält 3,0 g (27 % der Theorie) N'-(4,6-Dimethyl-pyrimidin-2-yl)-N"-benzyloxy-N"-(2-chlor-benzolsulfonyl)-guanidin vom Schmelzpunkt 159°C.

Analog Beispiel (IV-1) können die in der nachstehenden Tabelle 3 aufgeführten Verbindungen der Formel (IV) hergestellt werden:

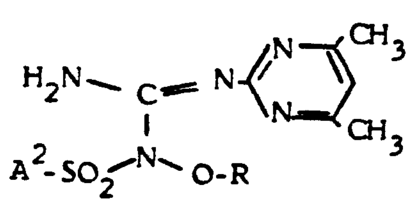

(IV)

Tabelle 3

| Beisp. Nr. | $A^2$ | R | Schmelz-punkt (°C) |
|---|---|---|---|
| IV-2 | 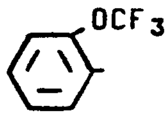 | $-CH_3$ | 111 |
| IV-3 | 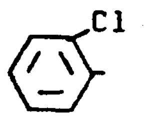 | $-CH_3$ | 97 |
| IV-4 | 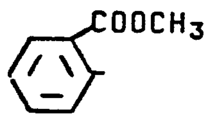 | $-CH_3$ | 161 |
| IV-5 | 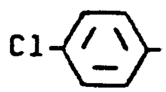 | $-CH_3$ | 90 |
| IV-6 | 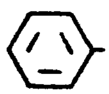 | $-CH_3$ | 101 |
| IV-7 | 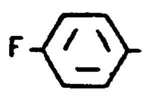 | $-CH_3$ | 106 |
| IV-8 | 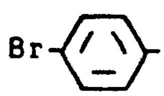 | $-CH_3$ | 112 |
| IV-9 | 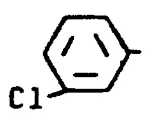 | $-CH_3$ | 98 |
| IV-10 | 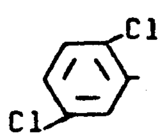 | $-CH_3$ | 162 |

Tabelle 3 - Fortsetzung

| Beisp. Nr. | $A^2$ | R | Schmelzpunkt (°C) |
|---|---|---|---|
| IV-11 | 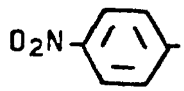 | $-CH_3$ | 121 |
| IV-12 | 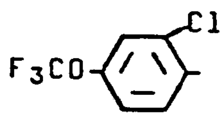 | $-CH_3$ | braunes Oel |
| IV-13 | 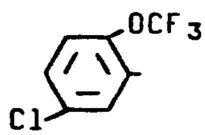 | $-CH_3$ | amorph |
| IV-14 | 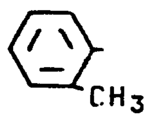 | $-CH_3$ | 91 |
| IV-15 | 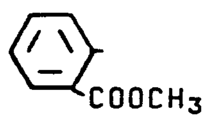 | 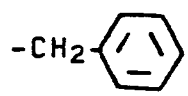 | glasartig |
| IV-16 | 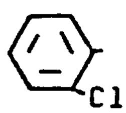 | $-C_4H_9$-n | 90 |
| IV-17 | 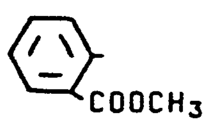 | $-C_8H_{17}$-n | glasartig |

Tabelle 3 - Fortsetzung

| Beisp. Nr. | $A^2$ | R | Schmelzpunkt (°C) |
|---|---|---|---|
| IV-18 | 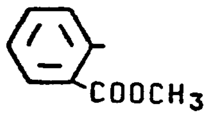 | $-C_4H_9-n$ | 95 |
| IV-19 | 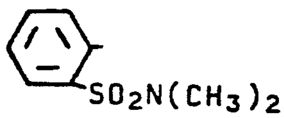 | $-CH_3$ | 116 |
| IV-20 | 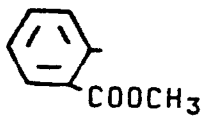 | 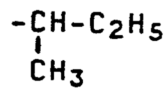 | 88 |
| IV-21 | 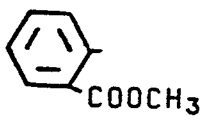 | $-C_2H_5$ | 115 |
| IV-22 | 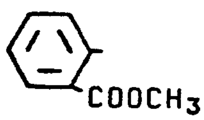 | $-CH_2-CH(CH_3)_2$ | 91 |
| IV-23 | 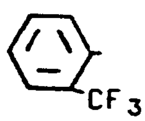 | $-C_2H_5$ | 90 |
| IV-24 | 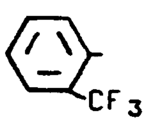 | $-CH_2-CH(CH_3)_2$ | 97 |

Tabelle 3 - Fortsetzung

| Beisp. Nr. | $A^2$ | R | Schmelzpunkt (°C) |
|---|---|---|---|
| IV-25 | 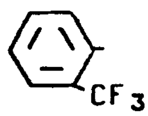 | $-CH-C_2H_5$<br>$\vert$<br>$CH_3$ | 113 |
| IV-26 | 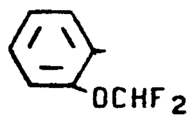 | $-CH_3$ | 130 (Zers.) |
| IV-27 | 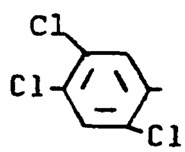 | $-CH_3$ | 137 |
| IV-28 | 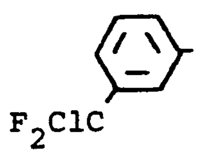 | $-CH_3$ | 82 |
| IV-29 | 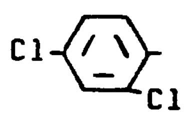 | $-CH_3$ | glasartig |
| IV-30 | 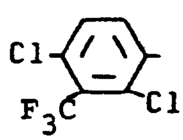 | $-CH_3$ | glasartig |
| IV-31 | 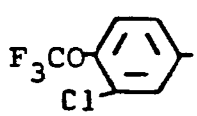 | $-CH_3$ | amorph |

Tabelle 3 - Fortsetzung

| Beisp. Nr. | $A^2$ | R | Schmelzpunkt (°C) |
|---|---|---|---|
| IV-32 | 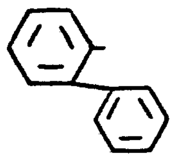 | $-CH_3$ | amorph |
| IV-33 | 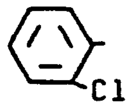 | $-CH_2-CH_2-CH_2-Cl$ | 92 (Zers.) |
| IV-34 | 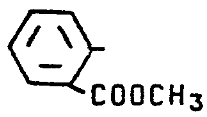 | $-CH_2-CH_2-CH_2-Cl$ | 110 - 111 |
| IV-35 | 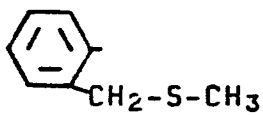 | $-CH_3$ | 92 - 93 |
| IV-36 | 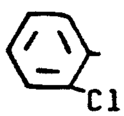 | $-CH_2-COOC_2H_5$ | 103 - 105 |
| IV-37 | 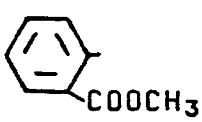 | $-CH_2-COOC_2H_5$ | 117 - 119 |
| IV-38 | 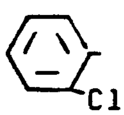 | 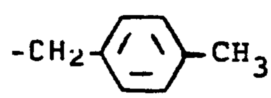 | |

Tabelle 3 - Fortsetzung

| Beisp. Nr. | $A^2$ | R | Schmelzpunkt (°C) |
|---|---|---|---|
| IV-39 | 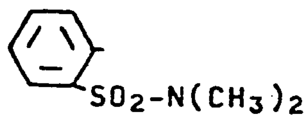 | $-CH_2CH(CH_3)_2$ | 116 |
| IV-40 | 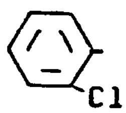 | $-CH_2-CH=CH_2$ | |
| IV-41 | 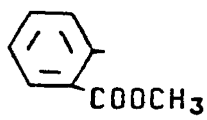 | 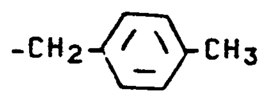 | |
| IV-42 | 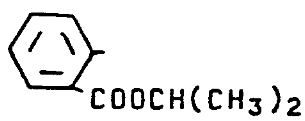 | $-CH_3$ | 174 |
| IV-43 | 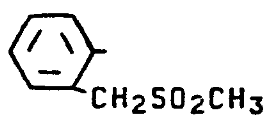 | $-CH_3$ | 115 - 116 |
| IV-44 | 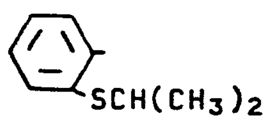 | $-CH_3$ | 93 |

Tabelle 3 - Fortsetzung

| Bsp.Nr. | $A^2$ | R | Schmelzpunkt (°C) |
|---|---|---|---|
| (IV-45) | 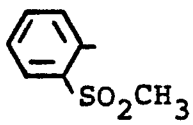 | $-CH_3$ | 148 |
| (IV-46) | 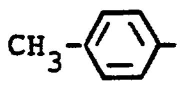 | $-C_4H_9$ | 114 |
| (IV-47) | 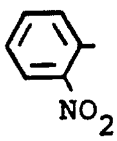 | $-CH_3$ | 135 |
| (IV-48) | 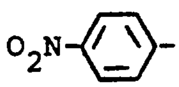 | $-C_3H_7$ | 132 |
| (IV-49) | 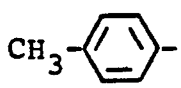 | $-CH_3$ | 93 |
| (IV-50) | 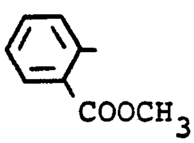 | 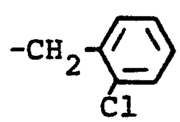 | 166 |
| (IV-51) | 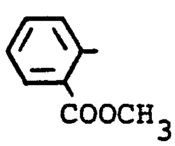 | 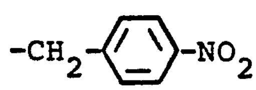 | 155 |
| (IV-52) | 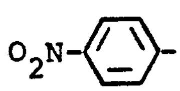 | 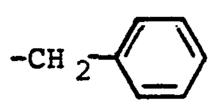 | |
| (IV-53) | 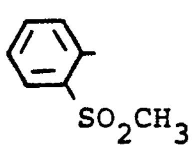 | $-C_8H_{17}$ | amorph |

Tabelle 3 - Fortsetzung

| Beisp.-Nr. | $A^2$ | R | Schmelzpunkt (°C) |
|---|---|---|---|
| (IV-54) | 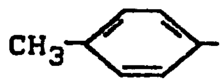 | $-C_8H_{17}$ | amorph |
| (IV-55) | $C_8F_{17}$- | $-CH_3$ | 63 |
| (IV-56) | $C_4H_9$- | 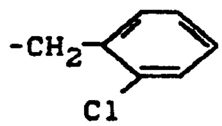 | 113 |
| (IV-57) | $(CH_3)_2N$- | $-CH_3$ | 147 |
| (IV-58) | $C_4H_9$- | $-CH_3$ | 92 -95 |
| (IV-59) | 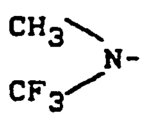 | $-CH_3$ | |
| (IV-60) | $CH_3$- | $-CH_3$ | 107 - 108 |
| (IV-61) | 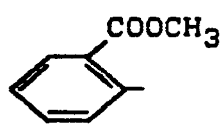 | $-C_3H_7$(-n) | 100 |
| (IV-62) | 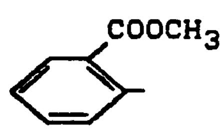 | $-C_3H_7$(-i) | 123 |

**Tabelle 3 - Fortsetzung**

| Beisp.-Nr. | $A^2$ | R | Schmelzpunkt (°C) |
|---|---|---|---|
| (IV-63) | 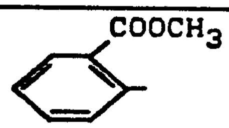 | $-CH_2-CH=CH_2$ | 102 |
| (IV-64) | 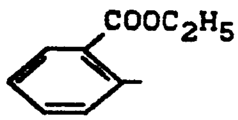 | $-CH_3$ | 125 |
| (IV-65) | 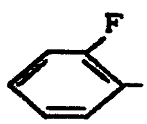 | $-CH_3$ | 117 |
| (IV-66) | 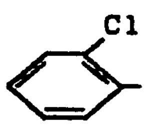 | $-C_2H_5$ | 114 |
| (IV-67) | 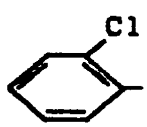 | $-CH_2-CH(CH_3)_2$ | 115 |
| (IV-68) | 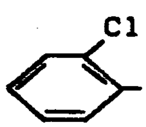 | $-\underset{CH_3}{\underset{\mid}{CH}}-C_2H_5$ | 125 |
| (IV-69) | 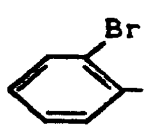 | $-CH_3$ | 104 |

Tabelle 3 - Fortsetzung

| Beisp.-Nr. | $A^2$ | R | Schmelzpunkt (°C) |
|---|---|---|---|
| (IV-70) | 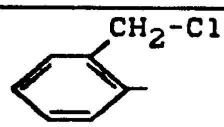 | $-CH_3$ | 101 |
| (IV-71) | 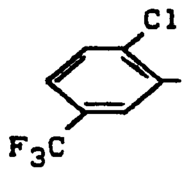 | $-CH_3$ | 134 |
| (IV-72) | 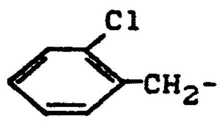 | $-CH_3$ | 164 - 166 |
| (IV-73) | $CH_3-$ | $-CH_2-CH(CH_3)_2$ | 107 |
| (IV-74) | $CH_3-$ | $-CH_2-COOC_2H_5$ | 89 - 90 |
| (IV-75) | $ClCH_2CH_2CH_2CH_2-$ | $-CH_3$ | 82 - 83 |
| (IV-76) | $(CH_3)_2N-$ | $-CH_2-COOC_2H_5$ | 87 - 89 |

Herstellung von Ausgangsstoffen der Formel (VI)

Beispiel (VI-1)

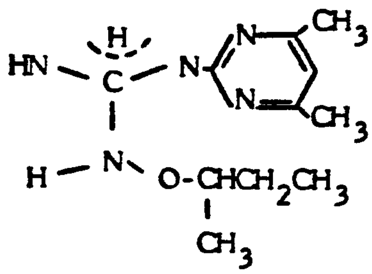

Eine Mischung aus 143 g (0,97 Mol) 2-Cyanamino-4,6-dimethyl-pyrimidin, 94,3 g (1,06 Mol) O-sec-Butyl-hydroxylamin und 190 ml Ethanol wird 6 Stunden unter Rückfluß zum Sieden erhitzt. Dann wird abgesaugt, das Filtrat eingeengt und der Rückstand mit 500 ml Wasser versetzt. Das hierbei kristallin angefallene Produkt wird durch Absaugen isoliert.

Man erhält 131 g (57 % der Theorie) N'-(4,6-Dimethyl-pyrimidin-2-yl)-N"-sec-butoxy-guanidin vom Schmelzpunkt 78°C.

Beispiel (VI-2)

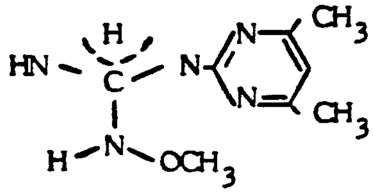

Eine Mischung aus 109 g (0,67 Mol) O-Methylhydroxylamin-Hydrochlorid, 99 g (0,67 Mol) 2-Cyanamino-4,6-dimethyl-

pyrimidin und 600 ml Ethanol wird 7 Stunden unter Rückfluß zum Sieden erhitzt. Anschließend wird der Alkohol im Wasserstrahlvakuum abdestilliert, der Rückstand in heißem Wasser gelöst und diese Lösung zu 100 ml konzentriertem Ammoniak gegeben. Das auskristallisierte Produkt wird abgesaugt und aus Ethanol umkristallisiert.

Man erhält 71,8 g (55 % der Theorie) N'-(4,6-Dimethyl-pyrimidin-2-yl)-N"-methoxy-guanidin vom Schmelzpunkt 134°C bis 136°C.

Analog können die in der nachstehenden Tabelle 4 aufgeführten Verbindungen der Formel (VI) hergestellt werden:

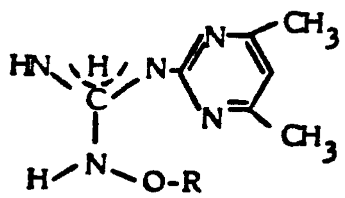

(VI)

Tabelle 4:

| Bsp. Nr. | R | Schmelzpunkt (°C) |
|---|---|---|
| VI-3 | $-CH_2CH(CH_3)_2$ | 52 |
| VI-4 | $-CH_2CH=CH_2$ | 103 |
| VI-5 | $-CH(CH_3)_2$ | 84 |
| VI-6 | $-CH_2CH_2-$ 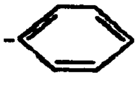 | $n_D^{24,5}$ = 1,5776 |

Tabelle 4 - Fortsetzung

| Beispiel Nr. | R | Schmelzpunkt (°C) |
|---|---|---|
| VI-7 | $-C_8H_{17}-n$ | 58 |
| VI-8 | 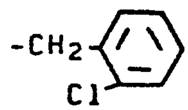 | 102 - 103 |
| VI-9 | $-CH_2CH_2CH_2Cl$ | 192 (Zers.) |
| VI-10 | 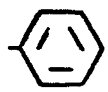 | Oel |
| VI-11 | $-CH_2-COOCH_3$ | 148 - 149 |
| VI-12 | $-CH_2-COOC_2H_5$ | 98 - 99 |
| VI-13 | $-\underset{\substack{\vert \\ CH_3}}{CH}-COOCH_3$ | 147 - 148 |
| VI-14 | 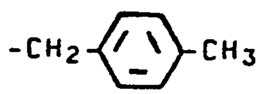 | 85 - 86 |
| VI-15 | 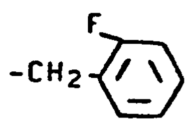 | 114 -116 |
| VI-16 | 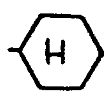 | |
| VI-17 | 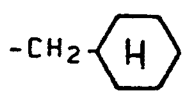 | |
| VI-18 | $-CH_2CON(CH_3)_2$ | |

Tabelle 4 - Fortsetzung

| Beispiel Nr. | R | Schmelzpunkt (°C) |
|---|---|---|
| VI-19 | $-CH_2OCH_3$ | |
| VI-20 | $-CH_2SCH_3$ | |
| VI-21 | 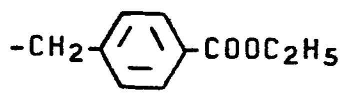 | 138 |
| VI-22 | $-CH_2CF_3$ | |
| VI-23 | 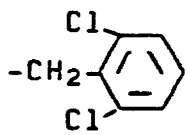 | 152 |
| VI-24 | 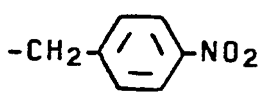 | 170 - 172 |
| VI-25 | $-C_4H_9$-n | $n_D^{20}$ = 1.5513 |
| VI-26 | $-C_3H_7$-n | 54 |

Tabelle 4 - Fortsetzung

| Beisp.-Nr. | R | Schmelzpunkt (°C) |
|---|---|---|
| VI-27 | $-C_2H_5$ | 88 |
| VI-28 | $-CH_2-CH_2-Cl$ | amorph |
| VI-29 | $-CH_2-CH_2-CH_2-CH_2-Cl$ | amorph |
| VI-30 | $-CH_2-COOH$ | 195 |
| VI-31 | $-CH_2-COOCH(CH_3)_2$ | 112 |
| VI-32 | $-CH_2$ 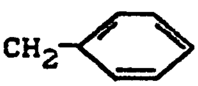 | 102 |
| VI-33 | -CH 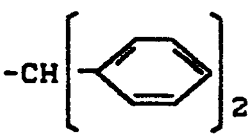 | 147 - 148 |

Herstellung von Verbindungen der Formel (IX) bzw. (XI)

Beispiel (IX-1)

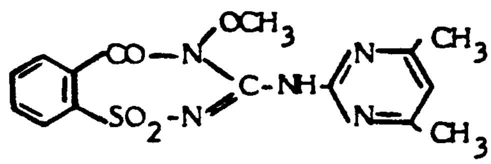

Eine Lösung von 60 g (0,25 Mol) 2-Chlorcarbonyl-benzolsulfonsäurechlorid in 100 ml Methylenchlorid wird zu einer auf -10°C gekühlten Mischung aus 49 g (0,25 Mol) N'-(4,6-Dimethyl-pyrimidin-2-yl)-N"-methoxy-guanidin, 50 g (0,63 Mol) Pyridin und 200 ml Methylenchlorid unter Rühren tropfenweise gegeben. Das Reaktionsgemisch wird 3 Stunden bei 20°C gerührt, dann zweimal mit je 200 ml 5 %iger Salzsäure und einmal mit 200 ml Wasser gewaschen, getrocknet, filtriert und eingeengt. Der Rückstand wird mit Methanol digeriert und das kristallin angefallene Produkt durch Absaugen isoliert.

Man erhält 65 g (72 % der Theorie) der Verbindung der oben angegebenen Strukturformel vom Schmelzpunkt 182°C.

Beispiel (XI-1)

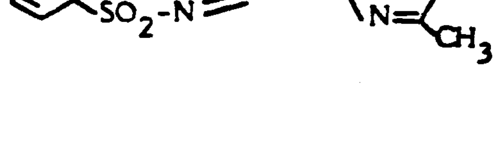

14 g (0,05 Mol) Benzol-1,2-disulfonsäuredichlorid werden zu einer auf -20°C gekühlten Mischung aus 10 g (0,05 Mol) N'-(4,6-Dimethyl-pyrimidin-2-yl)-N"-methoxy-guanidin, 12 g (0,05 Mol) Pyridin und 100 ml Methylenchlorid unter Rühren gegeben. Das Reaktionsgemisch wird 3 Stunden bei -20°C und weitere 15 Stunden bei +20°C gerührt, dann mit eiskalter 5 %iger Salzsäure und mit Wasser gewaschen, getrocknet, filtriert und eingeengt. Das beim Verreiben des Rückstandes kristallin angefallene Produkt wird durch Absaugen isoliert.

Man erhält 11,5 g (58 % der Theorie) der Verbindung der oben angegebenen Strukturformel vom Schmelzpunkt 158°C.

Analog können die in der nachstehenden Tabelle 5 aufgeführten Verbindungen der Formel (IX) bzw. (XI) hergestellt werden:

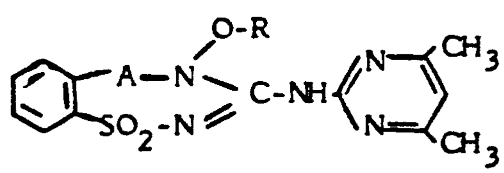

(IX) A=CO
(XI) A=$SO_2$

Tabelle 5

| Beisp. Nr. | A | R | Schmelzpunkt (°C) |
|---|---|---|---|
| XI-2 | $SO_2$ | $-C_4H_9-n$ | 179 |
| XI-3 | $SO_2$ | $-CH_2-$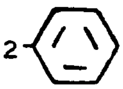 | 198 |
| IX-2 | CO | $-CH_2-$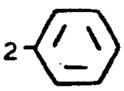 | 162 |
| IX-3 | CO | $-C_8H_{17}-n$ | 136 |
| IX-4 | CO | $-C_4H_9-n$ | (amorph) |
| XI-4 | $SO_2$ | $-C_8H_{17}-n$ | 164 |
| IX-5 | CO | $-C_3H_7-n$ | 81 |
| IX-6 | CO | $-CH(CH_3)_2$ | 155 |
| IX-7 | CO | $-C_2H_5$ | 175 |
| XI-5 | $SO_2$ | $-C_2H_5$ | 104 (Zers.) |
| XI-6 | $SO_2$ | $-C_3H_7(-i)$ | amorph |
| IX-8 | CO | $-CH_2-CH=CH_2$ | 153-156 |
| XI-7 | $SO_2$ | $-C_3H_7(-n)$ | 134 |
| XI-8 | $SO_2$ | $-CH_2-COOC_2H_5$ | 210 (Zers.) |
| XI-9 | $SO_2$ | $-CH_2-CH=CH_2$ | 180 (Zers.) |

**Beispiel A**

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Die erfindungsgemäßen Wirkstoffe zeigen in diesem Test eine sehr gute herbizide Wirksamkeit, insbesondere die Verbindungen der Herstellungsbeispiele 7, 8, 9, 10, 11 und 12.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Die erfindungsgemäßen Wirkstoffe zeigen in diesem Test eine sehr gute herbizide Wirksamkeit, insbesondere die Verbindungen der Herstellungsbeispiele 7, 8, 9, 10, 11 und 12.

## Patentansprüche

1) Unsymmetrische Sulfonylguanidine der allgemeinen Formel (I)

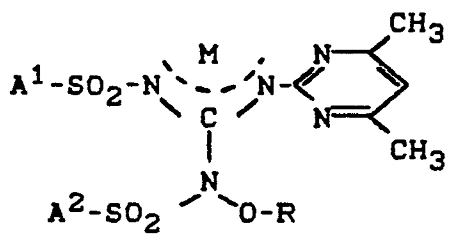

(I)

in welcher

die Reste $A^1$ und $A^2$ in jedem Einzelfall verschieden sind und jeweils einer der beiden Reste ($A^1$ oder $A^2$) für einen Rest der Formel

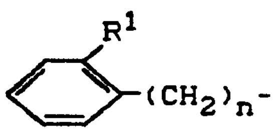

steht, worin

n für Null oder 1 steht und

$R^1$ für Halogen, Cyano, Nitro, $C_1$-$C_4$-Alkyl [welches gegebenenfalls durch Fluor, Chlor, Brom, Cyano, $C_1$-$C_4$-Alkoxy-carbonyl, $C_1$-$C_4$-Alkylamino-carbonyl, Di-($C_1$-$C_4$-alkyl)-amino-carbonyl,

$C_1$-$C_4$-Alkoxy, $C_1$-$C_4$-Alkylthio, $C_1$-$C_4$-Alkylsulfinyl oder $C_1$-$C_4$-Alkyl-sulfonyl substituiert ist], für $C_1$-$C_4$-Alkoxy [welches gegebenenfalls durch Fluor, Chlor, Brom, Cyano, $C_1$-$C_4$-Alkoxycarbonyl, $C_1$-$C_4$-Alkoxy, $C_1$-$C_4$-Alkylthio, $C_1$-$C_4$-Alkyl-sulfinyl oder $C_1$-$C_4$-Alkyl-sulfonyl substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor, Chlor, Brom, Cyano oder $C_1$-$C_4$-Alkoxy-carbonyl substituiert sind], für $C_1$-$C_4$-Alkylamino-sulfonyl, Di-($C_1$-$C_4$-alkyl)-amino-sulfonyl, $C_1$-$C_4$-Alkoxyamino-sulfonyl, Benzyloxy-aminosulfonyl, $C_1$-$C_4$-Alkoxy-$C_1$-$C_4$-alkyl-amino-sulfonyl, für Phenyl oder Phenoxy, für $C_1$-$C_4$-Akoxysulfonyl, für $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], für $C_2$-$C_6$-Alkinyl [welches gegebenenfalls durch Fluor, Chlor, Brom, Cyano oder $C_1$-$C_4$-Alkoxy-carbonyl substituiert ist], für $C_3$-$C_6$-Alkenoxy [welches gegebenenfalls durch Fluor, Chlor, Brom, Cyano oder $C_1$-$C_4$-Alkoxy-carbonyl substituiert ist], für $C_3$-$C_6$-Alkenylthio [welches gegebenenfalls durch Fluor, Chlor, Brom, Cyano oder $C_1$-$C_4$-Alkoxy-carbonyl substituiert ist], für $C_1$-$C_6$-Alkoxy-carbonyl [welches gegebenenfalls durch Fluor, Chlor, Brom, Cyano oder $C_1$-$C_4$-Alkoxy substituiert ist], für $C_3$-$C_6$-Cycloalkyloxy-carbonyl, für $C_1$-$C_6$-Alkylthio-carbonyl [welches gegebe-

nenfalls durch Fluor, Chlor, Brom, Cyano oder $C_1$-$C_4$-Alkoxy-carbonyl substituiert ist], für Aminocarbonyl, $C_1$-$C_4$-Alkylamino-carbonyl, Di-($C_1$-$C_4$-alkyl)-amino-carbonyl, $C_1$-$C_4$-Alkoxy-amino-carbonyl, $C_3$-$C_4$-Alkenoxyamino-carbonyl, Benzyloxyamino-carbonyl, $C_1$-$C_4$-Alkoxy-$C_1$-$C_4$-alkyl-amino-carbonyl, Dimethylhydrazino-carbonyl, oder Dimethylhydrazino-sulfonyl steht,

und in welcher jeweils der andere der beiden Reste ($A^2$ bzw. $A^1$)

für $C_1$-$C_{12}$-Alkyl [welches gegebenenfalls durch Fluor, Chlor, Brom, Cyano, Nitro, $C_1$-$C_4$-Alkoxy-carbonyl, $C_1$-$C_4$-Alkylamino-carbonyl, Di-($C_1$-$C_4$-alkyl)-amino-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist] für $C_1$-$C_{12}$-Alkoxy [welches gegebenenfalls durch Fluor, Chlor, Brom, Cyano, Nitro, $C_1$-$C_4$-Alkoxycarbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_2$-$C_{12}$-Alkenyl [welches gegebenenfalls durch Fluor, Chlor, Brom, Cyano, Nitro, $C_1$-$C_4$-Alkoxy, $C_1$-$C_4$-Alkylthio oder Phenyl substituiert ist], für $C_2$-$C_{12}$-Alkenyloxy [welches gegebenenfalls durch Fluor, Chlor, Brom, Cyano, Nitro, $C_1$-$C_4$-Alkoxy, $C_1$-$C_4$-Alkylthio oder Phenyl substituiert ist], für $C_1$-$C_8$-Alkylamino, $C_3$-$C_6$-Cycloalkylamino, Di-($C_3$-$C_6$-cycloalkyl)-

amino oder Di-($C_1$-$C_4$-alkyl)-amino steht [welche gegebenenfalls durch Fluor, Chlor, Brom, Cyano, Nitro, Phenyl, Phenoxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiert sind], für $C_2$-$C_8$-Alkenylamino [welches gegebenenfalls durch Fluor, Chlor, Brom, Cyano, Nitro oder Phenyl substituiert ist], für Phenylamino oder Benzylamino [welche gegebenenfalls durch Fluor, Chlor, Brom, $C_1$-$C_4$-Alkyl, Trifluormethyl, $C_1$-$C_4$-Alkoxy, $C_1$-$C_2$-Fluoralkoxy, $C_1$-$C_4$-Alkylthio, $C_1$-$C_4$-Alkylsulfinyl, $C_1$-$C_4$-Alkylsulfonyl, Cyano, Nitro und/oder $C_1$-$C_4$-Alkoxy-carbonyl substituiert sind], für Benzyl oder Phenyl steht, wobei letztere gegebenenfalls durch einen oder mehrere Reste aus der Reihe Halogen, Cyano, Nitro, $C_1$-$C_4$-Alkyl [welches gegebenenfalls durch Fluor, Chlor, Brom, Cyano, Carboxy, $C_1$-$C_4$-Alkoxy-carbonyl, $C_1$-$C_4$-Alkyl-amino-carbonyl, $C_1$-$C_4$-Alkoxy-amino-carbonyl, Di-($C_1$-$C_4$-alkyl)-amino-carbonyl, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkyl-amino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl oder Phenyl substituiert ist], $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], $C_2$-$C_6$-Alkinyl [welches gegebenenfalls durch Fluor, Chlor, Brom, Cyano, $C_1$-$C_4$-Alk-

oxy-carbonyl oder Phenyl substituiert ist], $C_1$-$C_4$-Alkoxy [welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist], $C_3$-$C_6$-Alkenoxy [welches gegebenenfalls durch Fluor, Chlor, Brom, Cyano oder $C_1$-$C_4$-Alkoxy-carbonyl substituiert ist], $C_3$-$C_6$-Alkenylthio [welches gegebenenfalls durch Fluor, Chlor, Brom, Cyano oder $C_1$-$C_4$-Alkoxy-carbonyl substituiert ist], $C_1$-$C_4$-Alkylthio, $C_1$-$C_4$-Alkylsulfinyl oder $C_1$-$C_4$-Alkylsulfonyl [welche gegebenenfalls durch Fluor, Chlor, Brom, Cyano oder $C_1$-$C_4$-Alkoxy-carbonyl substituiert sind], Phenyl, Phenoxy, Di-($C_1$-$C_4$-alkyl)-amino-sulfonyl, $C_1$-$C_4$-Alkyl-amino-sulfonyl, $C_1$-$C_4$-Alkoxy-$C_1$-$C_4$-alkyl-amino-sulfonyl, $C_1$-$C_4$-Alkoxysulfonyl, $C_1$-$C_4$-Alkoxy-amino-sulfonyl, $C_1$-$C_6$-Alkoxy-carbonyl, $C_3$-$C_6$-Cycloalkoxy-carbonyl, $C_1$-$C_6$-Alkylamino-carbonyl, Di-($C_1$-$C_4$-alkyl)-amino-carbonyl, $C_1$-$C_6$-Alkoxy-amino-carbonyl, $C_1$-$C_4$-Alkoxy-$C_1$-$C_4$-alkyl-amino-carbonyl, $C_1$-$C_6$-Alkylaminosulfonylamino oder Di-($C_1$-$C_6$-alkyl)-amino-sulfonylamino substituiert sind und/oder gegebenenfalls benzanelliert sind:

in welcher weiter

R für $C_1$-$C_{12}$-Alkyl [welches gegebenenfalls durch Fluor, Chlor, $C_1$-$C_4$-Alkoxy, $C_1$-$C_4$-Alkylthio, $C_1$-$C_4$-Alkylsulfinyl, $C_1$-$C_4$-Alkylsulfonyl, Cyano oder Nitro substituiert ist], $C_3$-$C_6$-Alkenyl

[welches gegebenenfalls durch Fluor oder Chlor substituiert ist], $C_3$-$C_6$-Alkinyl, $C_1$-$C_4$-Alkoxy-carbonyl-$C_1$-$C_2$-alkyl, Aminocarbonyl-$C_1$-$C_2$-alkyl, $C_1$-$C_4$-Alkyl-amino-carbonyl-$C_1$-$C_2$-alkyl, Di-($C_1$-$C_4$-alkyl)-amino-carbonyl-$C_1$-$C_2$-alkyl, für Phenyl, Phenylethyl, Benzhydryl oder Benzyl [welche gegebenenfalls durch Fluor, Chlor, Brom, Cyano, Nitro, $C_1$-$C_4$-Alkyl, $C_1$-$C_4$-Alkoxy, Carboxy oder $C_1$-$C_4$-Alkoxy-carbonyl substituiert sind] steht und

M für Wasserstoff oder ein Metalläquivalent steht,

sowie 1:1-Addukte von Verbindungen der Formel (I) mit starken Säuren,

wobei folgende Verbindungen ausgenommen sind:

N'-(4,6-Dimethyl-pyrimidin-2-yl)-N"-methoxy-N"-(2-chlor-benzolsulfonyl)-N"'-(2-methoxycarbonyl-benzolsulfonyl)-guanidin,
N'-(4,6-Dimethyl-pyrimidin-2-yl)-N"-methoxy-N"-(3-chlor-benzolsulfonyl)-N"'-(2-methoxycarbonyl- benzolsulfonyl)-guanidin und
N'-(4,6-Dimethyl-pyrimidin-2-yl)-N"-methoxy-N"-(4-methyl-benzolsulfonyl)-N"'-(2-methoxycarbonyl-benzolsulfonyl)-guanidin.

2) Verfahren zur Herstellung von unsymmetrischen Sulfonylguanidinen der allgemeinen Formel (I) gemäß Anspruch 1, dadurch gekennzeichnet, daß man

(a) für den Fall, daß M für Wasserstoff steht, Sulfonylguanidine der Formel (II)

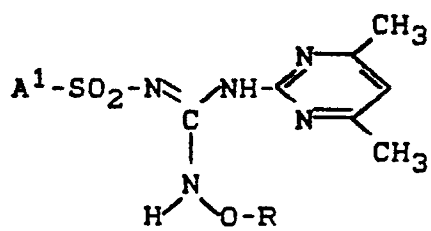

(II)

in welcher

$A^1$ und R die oben angegebenen Bedeutungen haben,

mit Sulfonsäurechloriden der Formel (III)

$$A^2\text{-}SO_2\text{-}Cl \qquad (III)$$

in welcher

$A^2$ die oben angegebene Bedeutung hat,

gegebenenfalls in Gegenwart von Säureakzeptoren und gegebenenfalls in Gegenwart von Verdünnungsmitteln umsetzt;

oder daß man

(b) für den Fall, daß M für Wasserstoff steht, Sulfonylguanidine der Formel (IV)

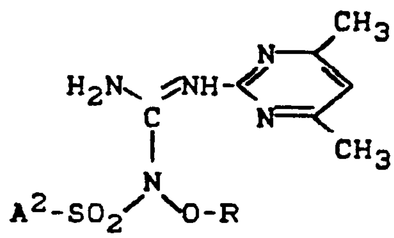

(IV)

in welcher

$A^2$ und R die oben angegebenen Bedeutungen haben,

mit Sulfonsäurechloriden der Formel (V)

$$A^1\text{-}SO_2Cl \qquad (V)$$

in welcher

$A^1$ die oben angegebene Bedeutung hat,

gegebenenfalls in Gegenwart von Säureakzeptoren und gegebenenfalls in Gegenwart von Verdünnungsmitteln umsetzt:

oder daß man

(c) für den Fall, daß M für ein Metalläquivalent steht, die nach dem oben unter (a) und (b) angegebenen Verfahren erhältlichen Verbindungen

der Formel (I), in welcher M für Wasserstoff steht und R sowie $A^1$ und $A^2$ die oben angegebenen Bedeutungen haben, mit Metall-hydroxiden, -hydriden oder -alkanolaten oder mit metallorganischen Verbindungen, gegebenenfalls in Gegenwart von Verdünnungsmitteln umsetzt;

oder daß man

(d) für den Fall, daß 1:1-Addukte von Verbindungen der Formel (I) mit starken Säuren herzustellen sind,

Verbindungen der Formel (I), in welcher M für Wasserstoff steht und R sowie $A^1$ und $A^2$ die oben angegebene Bedeutung haben, mit starken Säuren gegebenenfalls in Gegenwart von inerten Verdünnungsmitteln umsetzt.

3) Herbizide Mittel, gekennzeichnet durch einen Gehalt an mindestens einem unsymmetrischen Sulfonylguanidin der allgemeinen Formel (I) gemäß Anspruch 1.

4) Verwendung von unsymmetrischen Sulfonylguanidinen der allgemeinen Formel (I) gemäß Anspruch 1 zur Bekämpfung von unerwünschtem Pflanzenwachstum.

5) Verfahren zur Herstellung von herbziden Mitteln, dadurch gekennzeichnet, daß man unsymmetrische Sulfonylguanidine der allgemeinen Formel (I) gemäß Anspruch 1 mit Streckmitteln und/oder oberflächenaktiven Mitteln vermischt.

Europäisches Patentamt

**EUROPÄISCHER RECHERCHENBERICHT**

**0 173 317**

| EINSCHLÄGIGE DOKUMENTE | | | EP 85110828.2 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
| P,A | EP - A1 - 0 121 082 (BAYER)<br>* Ansprüche 1-7 *<br>-- | 1-5 | C 07 D 239/42<br>A 01 N 47/44 |
| A | EP - A1 - 1 170 014 (DU PONT)<br>* Zusammenfassung *<br>-- | 1,3,4 | |
| A | US - A - 4 451 463 (LARGE)<br>* Anspruch 1 *<br>-- | 1 | |
| A | DE - A1 - 3 243 533 (SANDOZ)<br>* Anspruch 1,7 *<br>-- | 1,3 | |
| A | DD - A - 71 015 (KOCHMANN)<br>* Anspruch *<br>---- | 3 | RECHERCHIERTE SACHGEBIETE (Int. Cl.4)<br>C 07 D 239/00 |

Der vorliegende Recherchenbericht wurde für alle Patentansprüche erstellt.

| Recherchenort | Abschlußdatum der Recherche | Prüfer |
|---|---|---|
| WIEN | 17-10-1985 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82